# EUROPEAN PATENT APPLICATION

(11) **EP 2 407 464 A1**
(43) Date of publication of application: **18.01.2012**
(21) Application number: 10750871.5
(22) Date of filing: 10.03.2010
(51) Int. Cl.: C07D 333/78, H01L 29/786, H01L 29/80, H01L 51/05, H01L 51/30, H01L 51/42

(54) **BRANCHED COMPOUND, AND ORGANIC THIN FILM AND ORGANIC THIN FILM ELEMENT EACH COMPRISING SAME**

(30) Priority: 11.03.2009 JP 2009058664
(71) Applicant: Sumitomo Chemical Company, Limited, Tokyo 104-8260 (JP); Osaka University, Suita-shi Osaka 565-0871 (JP)
(72) Inventor: IE Yutaka, Suita-shi Osaka 565-0871 (JP); OKABE Makoto, Suita-shi Osaka 565-0871 (JP); ASO Yoshio, Suita-shi Osaka 565-0871 (JP); UEDA Masato, Tsukuba-shi Ibaraki 305-0046 (JP)
(74) Representative: Duckworth, Timothy John
(86) International application number: PCT/JP2010/054015
(87) International publication number: WO 2010/104118

(57) **Abstract**

A branched compound having a construction with a core portion, at least 3 side chain portions bonded to the core portion, and end portions bonded to each of the side chain portions, wherein the side chain portions are groups in which a plurality of conjugated units are linked, at least one of the conjugated units being a divalent heterocyclic group, at least one of the end portions is a group represented by formula (1), and the side chain portions and the end portions are conjugated with the core portion. 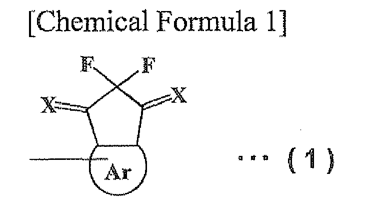 [In the formula, Ar represents a trivalent aromatic hydrocarbon or a trivalent heterocyclic group, and X represents oxygen, sulfur, or a group represented by formula (a).] 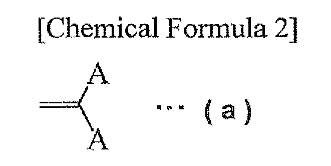 [In the formula, A represents hydrogen, a halogen or a monovalent group, and at least one A group is an electron-withdrawing group.]

## Description

### Technical Field

The present invention relates to a branched compound, and to an organic thin-film and an organic thin-film element comprising it.

### Background Art

A variety of conjugated compounds have been developed as organic n-type semiconductors, for use as materials in organic thin-film elements such as organic transistors, organic solar cells and optical sensors. Specific ones that have been proposed include compounds having fluoroalkyl groups introduced at the ends of oligothiophenes (Patent document 1).

### Citation List

### Patent Literature

[Patent document 1] International Patent Publication No. WO2003/010778

### Summary of Invention

### Technical Problem

The compounds mentioned above, however, cannot be utilized as organic n-type semiconductors with satisfactory electron transport properties.

It is therefore an object of the present invention to provide a novel branched compound that can be used as an organic n-type semiconductor with excellent electron transport properties. It is another object of the invention to provide an organic thin-film comprising the novel branched compound, and an organic thin-film element such as an organic thin-film transistor, organic solar cell or optical sensor, comprising the organic thin-film.

### Solution to Problem

In order to achieve the aforestated object, the invention provides a branched compound having a construction with a core portion, at least 3 side chain portions bonded to the core portion, and end portions bonded to each of the side chain portions, wherein the side chain portions are groups in which a plurality of conjugated units are linked, at least one of the conjugated units being a divalent heterocyclic group, at least one of the end portions is a group represented by formula (1), and the side chain portions and the end portions are conjugated with the core portion.

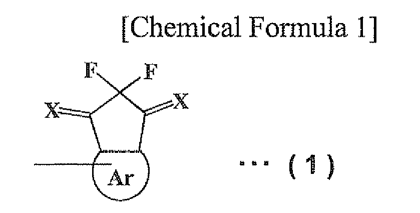

Ar represents an optionally substituted trivalent aromatic hydrocarbon or optionally substituted trivalent heterocyclic group, and X represents an oxygen atom, a sulfur atom, or a group represented by formula (a). When multiple X groups are present, they may be the same or different.

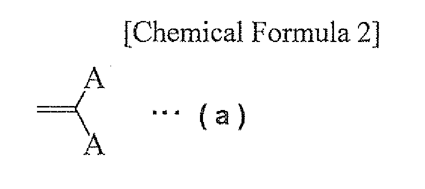

In the formula, A represents hydrogen, a halogen atom or a monovalent group, and when multiple A groups are present, they may be the same or different, and at least one A is an electron-withdrawing group.

Since the branched compound of the invention includes a heterocyclic structure in its 3 or more side chain portions and has the side chain portions and end portions conjugated with the core portion, the conjugation extension occurs in a planar or three-dimensional manner, and interaction between molecules is facilitated. Furthermore, since the ends have electron-withdrawing groups represented by formula (1) that include fluorine, it can have a sufficiently low LUMO. The branched compound is therefore sufficiently suitable as an n-type semiconductor with excellent electron injection and electron transport properties. Furthermore, because such a compound has a structure represented by ">C=X" adjacent to the fluorine-bonded carbon atom, in the electron-withdrawing groups, it is chemically stable and has excellent solubility in solvents, and can thus form organic thin-films that are homogeneous over large areas. By forming an organic thin-film using the branched compound, therefore, it is possible to produce an organic thin-film element with excellent performance.

From the viewpoint of allowing the LUMO to be even lower, the group represented by formula (1) in the branched compound is preferably a group represented by formula (2).

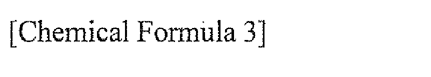

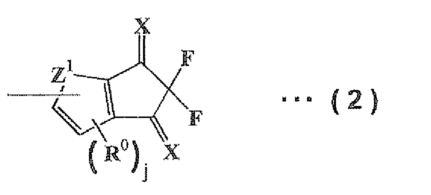

Here, X has the same definition as above, R⁰ represents hydrogen or a monovalent group, and j is an integer from 1 to the number of substitutable sites on the ring to which R⁰ is bonded. When multiple R⁰ groups are present, they may be the same or different. Z¹ represents any group represented by formula (i), (ii), (iii), (iv), (v), (vi), (vii), (viii) or (ix) (hereunder also referred to as "(i)-(ix)"), among which groups R¹, R², R³ and R⁴ may be the same or different and each represents hydrogen or a monovalent group, and R¹ and R² may be bonded together to form a ring.

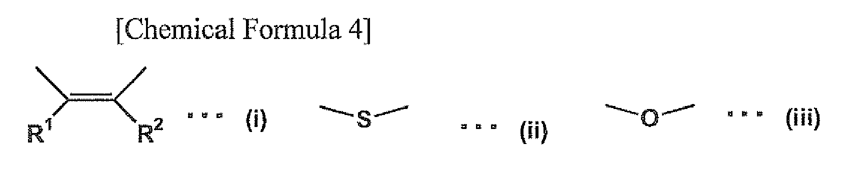

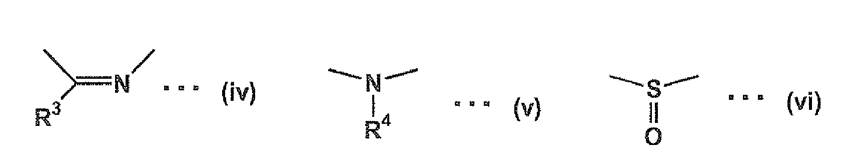

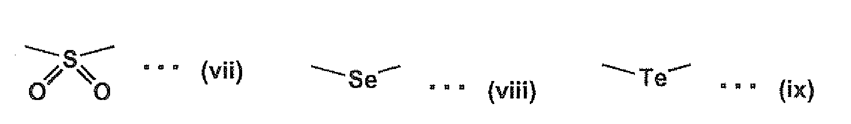

In the group represented by formula (2), Z¹ is preferably a group represented by formula (ii). Such a conjugated compound has a sufficiently low LUMO and a more excellent electron transport property. It can therefore suitably be used as an organic n-type semiconductor.

In the branched compound described above, the side chain portions are preferably groups represented by formula (3).

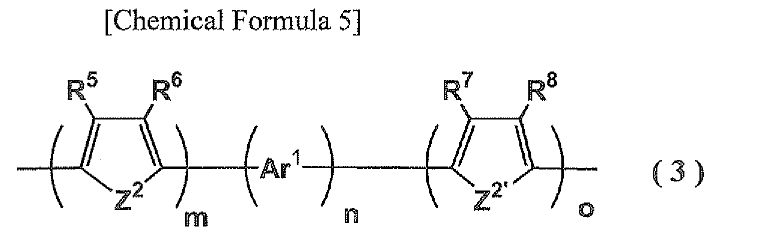

In the formula, m, n and o are the same or different and each represents an integer of 0-10, with the proviso that m+o is an integer of 1 or greater. Ar¹ represents an optionally substituted divalent aromatic hydrocarbon or an optionally substituted divalent heterocyclic group, and R⁵, R⁶, R⁷ and R⁸ are the same or different and each represents hydrogen, alkyl, alkoxy, optionally substituted aryl or an optionally substituted monovalent heterocyclic group. The groups represented by Ar¹, R⁵, R⁶, R⁷ and R⁸ may have all or a portion of their hydrogens replaced by fluorine. Z² and Z^{2'} are the same or different and each is a group represented by one of formulas (xi)-(xix), wherein R⁹, R¹⁰, R¹¹ and R¹² are the same or different and each represents hydrogen or a monovalent group, and R⁹ and R¹⁰ may be bonded together to form a ring. When multiple Z², Z^{2'}, Ar¹, R⁵, R⁶, R⁷, R⁸, R⁹, R¹⁰, R¹¹ and R¹² groups are present, they may be the same or different.

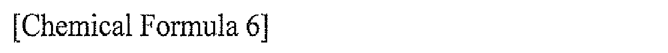

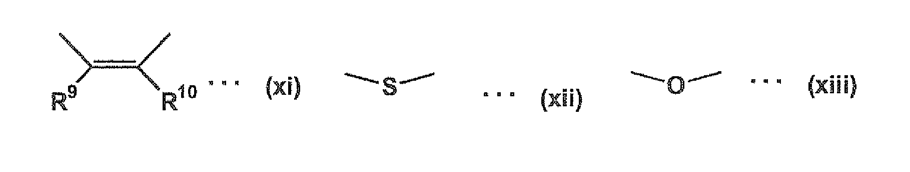

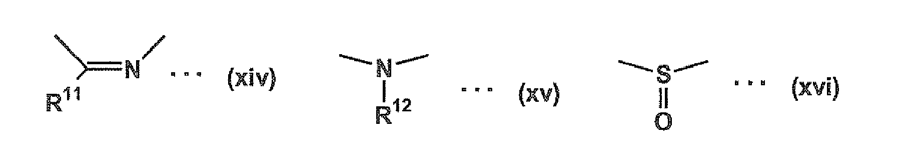

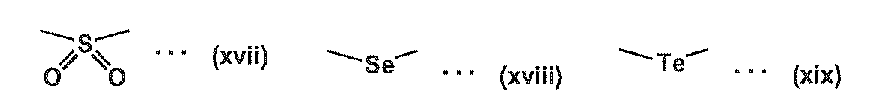

In formula (3), either or both Z² and Z^{2'} are preferably a group represented by formula (xii).

A branched compound in which the side chain portions have the structure described above exhibits even more notable interaction between molecules, has an even lower LUMO, and can be even more suitably used as an n-type semiconductor with an excellent electron transport property.

The core portions in the branched compound are preferably any group represented by formula (I), (II), (III), (IV) or (V).

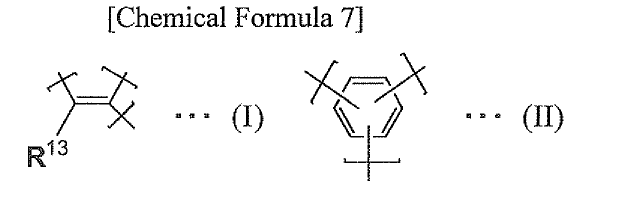

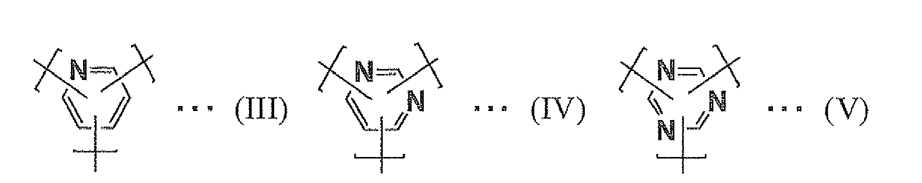

In the formula, R¹³ represents hydrogen, alkyl, aryl or cyano.

A branched compound having a core portion with the structure described above facilitates conjugation between the side chain portions and the core portion, and conjugation extension tends to be planar or three-dimensional.

The invention further provides an organic thin-film element, an organic thin-film transistor, an organic solar cell and an optical sensor comprising an organic thin-film containing the branched compound.

Because such an organic thin-film, organic thin-film element, organic thin-film transistor, organic solar cell or optical sensor is formed using a branched compound of the invention exhibiting an excellent electron transport property as mentioned above, it is possible to obtain excellent performance.

### Advantageous Effects of Invention

According to the invention it is possible to provide novel branched compounds that can be used as organic n-type semiconductors with an excellent electron transport property. Also, according to the invention it is possible to provide organic thin-films containing the branched compounds, and organic thin-film elements comprising the organic thin-films. Because the organic thin-film element comprises an organic thin-film of the invention, it can exhibit an excellent charge transport property and can also exhibit superior stability.

### Brief Description of Drawings

Fig. 1 is a schematic cross-sectional view of an organic thin-film transistor according to a first embodiment.
Fig. 2 is a schematic cross-sectional view of an organic thin-film transistor according to a second embodiment.
Fig. 3 is a schematic cross-sectional view of an organic thin-film transistor according to a third embodiment.
Fig. 4 is a schematic cross-sectional view of an organic thin-film transistor according to a fourth embodiment.
Fig. 5 is a schematic cross-sectional view of an organic thin-film transistor according to a fifth embodiment.
Fig. 6 is a schematic cross-sectional view of an organic thin-film transistor according to a sixth embodiment.
Fig. 7 is a schematic cross-sectional view of an organic thin-film transistor according to a seventh embodiment.
Fig. 8 is a schematic cross-sectional view of a solar cell according to an embodiment of the invention.
Fig. 9 is a schematic cross-sectional view of an optical sensor according to a first embodiment.
Fig. 10 is a schematic cross-sectional view of an optical sensor according to a second embodiment.
Fig. 11 is a schematic cross-sectional view of an optical sensor according to a third embodiment.

### Description of Embodiments

Preferred embodiments of the invention will now be explained in detail, with reference to the accompanying drawings as necessary. Throughout the drawings, corresponding elements will be referred to by like reference numerals and will be explained only once. Unless otherwise specified, the vertical and horizontal positional relationships are based on the positional relationships in the drawings. Also, the dimensional proportions depicted in the drawings are not necessarily limitative.

### (Branched compound)

The branched compound of this embodiment is a branched compound having a construction with a core portion, at least 3 side chain portions bonded to the core portion, and end portions bonded to each of the side chain portions, wherein the side chain portions are groups in which a plurality of conjugated units are linked, at least one of the conjugated units being a divalent heterocyclic group, at least one of the end portions is a group represented by formula (1), and the side chain portions and end portions are conjugated with the core portion. The core portion is preferably an organic group with a value of x (where x is an integer of 3 or greater and corresponds to the number of side chain portions, same hereunder).

At least one of the end portions of the branched compound of this embodiment is a group represented by formula (1), and preferably all of the end portions are groups represented by formula (1). The branched compound comprising such end portions has satisfactory conjugation, excellent compound stability, and a sufficient low LUMO. It therefore has a superior electron transport property and exhibits excellent properties when used as an organic thin-film element.

In formula (1), Ar represents an optionally substituted trivalent aromatic hydrocarbon or optionally substituted trivalent heterocyclic group, and X represents an oxygen atom, a sulfur atom, or a group represented by formula (a). X is preferably an oxygen atom or a group represented by formula (a), and more preferably an oxygen atom. Since the group represented by formula (1) has a specific structure containing fluorine, it exhibits an electron-withdrawing property and the branched compound comprising the group has a sufficiently low LUMO.

In formula (1), the trivalent aromatic hydrocarbon group represented by Ar is an atomic group remaining after removing 3 hydrogen atoms from a benzene ring or fused ring, and it is preferably a C6-60 or more preferably a C6-20 group. Fused rings include naphthalene, anthracene, tetracene, pentacene, pyrene, perylene, rubrene and fluorene rings. A trivalent aromatic hydrocarbon group is preferably an atomic group remaining after removing 3 hydrogen atoms from a benzene ring or a fluorene ring. The trivalent aromatic hydrocarbon groups may be optionally substituted. The numbers of carbon atoms of the substituents are not included in the number of carbon atoms in the trivalent aromatic hydrocarbon groups. Substituents include halogen atoms and saturated or unsaturated hydrocarbon, aryl, alkoxy, aryloxy, monovalent heterocyclic, amino, nitro and cyano groups.

A trivalent heterocyclic group represented by Ar is an atomic group remaining after removing 3 hydrogens from a heterocyclic compound, and it is preferably a C3-60 or more preferably a C3-20 group. Heterocyclic compounds include thiophene, thienothiophene, dithienothiophene, pyrrole, pyridine, pyrimidine, pyrazine, triazine, benzothiazole and benzothiadiazole. A trivalent heterocyclic group is preferably an atomic group remaining after removing 3 hydrogens from thiophene or thienothiophene. The trivalent heterocyclic group may be optionally substituted. The carbons of the substituents are not included in the number of carbon atoms of the trivalent heterocyclic group. Substituents include halogen atoms and saturated or unsaturated hydrocarbon, aryl, alkoxy, aryloxy, monovalent heterocyclic, amino, nitro and cyano groups.

In formula (a), A represents hydrogen, a halogen atom or a monovalent group. When multiple A groups are present, they may be the same or different, and at least one A group is an electron-withdrawing group, while from the viewpoint of allowing an even lower LUMO, preferably all of the A groups are electron-withdrawing groups. Examples of electron-withdrawing groups include cyano, nitro, aldehyde, acyl, alkoxycarbonyl, carboxyl, hydroxyl and halogen atoms, with cyano, nitro and halogen atoms being preferred, and cyano groups being especially preferred.

The group represented by formula (1) exhibits an electron-withdrawing property since it contains fluorine, and having such groups on the end portions facilitates interaction between the electron-withdrawing groups of different molecules and results in a sufficiently low LUMO. The electron-accepting property can often be Particularly increased when X is a group represented by formula (a). Furthermore, since the side chain portions and end portions are conjugated with the core portion, i.e. the core portion, side chain portions and end portions are conjugated as a whole, the branched compound functions as an organic n-type semiconductor with an excellent electron transport property.

End portions in the branched compound other than groups represented by formula (1) may be hydrogen or monovalent groups. Such monovalent groups are preferably alkyl, alkoxy, phenyl and substituted phenyl groups. Substituents include halogen atoms and saturated or unsaturated hydrocarbon, aryl, alkoxy, aryloxy, monovalent heterocyclic, amino, nitro and cyano groups. Some or all of the hydrogens of these groups may be replaced by fluorine. From the viewpoint of stability of the branched compound, phenyl and substituted phenyl groups are more preferred, and phenyl groups are even more preferred.

The groups represented by formula (1) are preferably groups represented by formula (2).

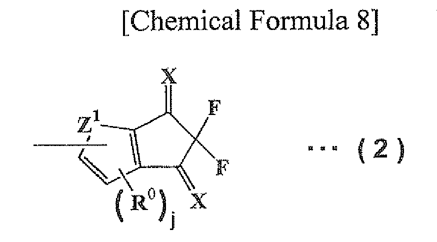

In formula (2), X has the same definition as above, R⁰ represents hydrogen or a monovalent group, and j is an integer from 1 to the number of substitutable sites on the ring to which R⁰ is bonded. When multiple R⁰ groups are present, they may be the same or different. Z¹ represents a group represented by one of formulas (i)-(ix), wherein R¹, R², R³ and R⁴ are the same or different and each represents hydrogen or a monovalent group, and R¹ and R² may be bonded together to form a ring.

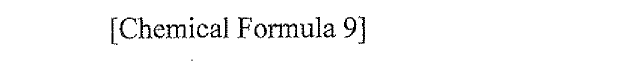

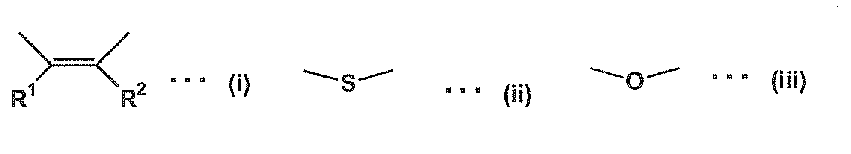

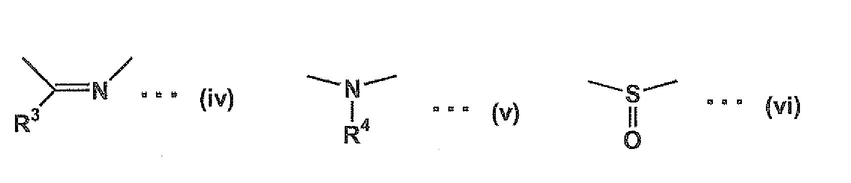

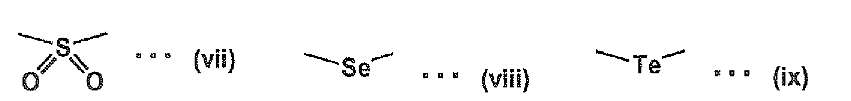

Monovalent groups represented by R⁰, R¹, R², R³ and R⁴ are preferably alkyl, alkoxy, optionally substituted aryl or optionally substituted monovalent heterocyclic groups, and some or all of the hydrogens in these groups may be replaced by fluorine. The same groups may be mentioned as for the monovalent groups represented by A. Also, Z¹ is preferably a group represented by formula (ii).

The side chain portions of the branched compound of this embodiment are groups in which multiple conjugated units are linked, having divalent heterocyclic groups as the conjugated units, and most preferably they have thienylene groups as the divalent heterocyclic groups.

Such side chain portions are preferably groups represented by formula (3).

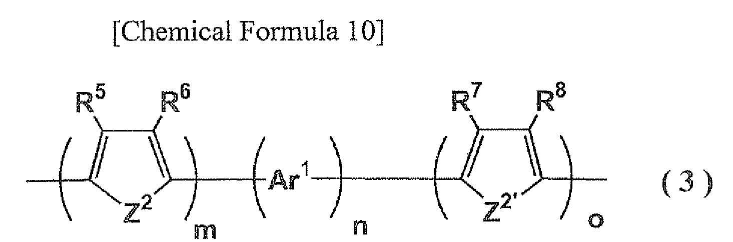

In the formula, m, n and o are the same or different and each represents an integer of 0-10. This is with the proviso that m+o is an integer of 1 or greater. Ar¹ represents an optionally substituted divalent aromatic hydrocarbon or an optionally substituted divalent heterocyclic group, and R⁵, R⁶, R⁷ and R⁸ are the same or different and each represents hydrogen, alkyl, alkoxy, optionally substituted aryl or an optionally substituted monovalent heterocyclic group. The groups represented by Ar¹, R⁵, R⁶, R⁷ and R⁸ may have all or a portion of their hydrogens replaced by fluorine. Z² and Z^{2'} are the same or different and each is a group represented by one of formulas (xi)-(xix), wherein R⁹, R¹⁰, R¹¹ and R¹² are the same or different and each represents hydrogen or a monovalent group, and R⁹ and R¹⁰ may be bonded together to form a ring. When multiple Z², Z^{2'}, Ar¹, R⁵, R⁶, R⁷, R⁸, R⁹, R¹⁰, R¹¹ and R¹² groups are present, they may be the same or different. However, since the side chain portions contain at least one divalent heterocyclic group, at least one of Z² and Z^{2'} will be a group represented by one of formulas (xii)-(xix).

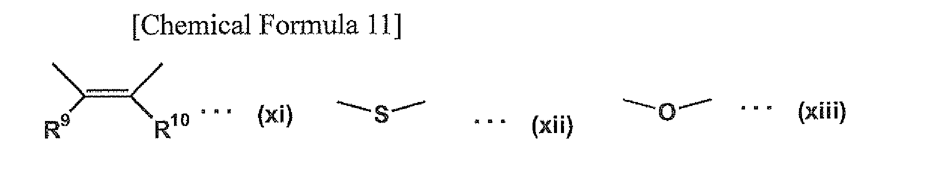

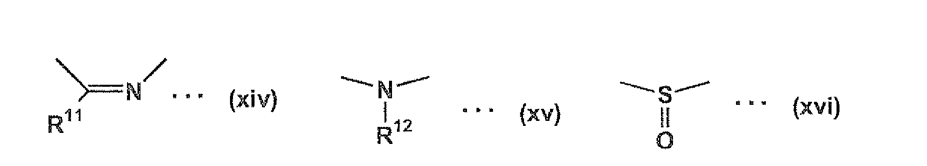

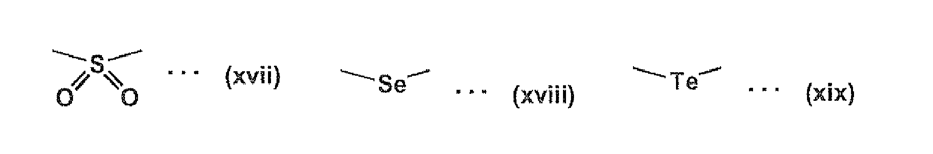

In formula (3), m, n and o are the same or different and are each preferably an integer of 0-6 and more preferably an integer of 0-3. In particular, m+n+o is preferably an integer of no greater than 6.

Monovalent groups represented by R⁹, R¹⁰, R¹¹ and R¹² in formula (3) are preferably alkyl, alkoxy, optionally substituted aryl or optionally substituted monovalent heterocyclic groups, and some or all of the hydrogens in these groups may be replaced by fluorine.

In formula (3), the divalent aromatic hydrocarbon group represented by Ar¹ is an atomic group remaining after removing 2 hydrogen atoms from a benzene ring or fused ring, and it is preferably a C6-60 or more preferably a C6-20 group. Fused rings include naphthalene, anthracene, tetracene, pentacene, pyrene, perylene, rubrene and fluorene rings. A divalent aromatic hydrocarbon group is preferably an atomic group remaining after removing 2 hydrogen atoms from a benzene ring or a fluorene ring. The divalent aromatic hydrocarbon groups may be optionally substituted. The numbers of carbon atoms of the substituents are not included in the number of carbon atoms in the divalent aromatic hydrocarbon groups. Substituents include halogen atoms and saturated or unsaturated hydrocarbon, aryl, alkoxy, aryloxy, monovalent heterocyclic, amino, nitro and cyano groups.

A divalent heterocyclic group represented by Ar¹ is an atomic group remaining after removing 2 hydrogens from a heterocyclic compound, and it is preferably a C3-60 or more preferably a C3-20 group. Heterocyclic compounds include thiophene, thienothiophene, dithienothiophene, pyrrole, pyridine, pyrimidine, pyrazine, triazine, benzothiazole and benzothiadiazole. A divalent heterocyclic group is preferably an atomic group remaining after removing 2 hydrogens from thiophene or thienothiophene. The divalent heterocyclic group may have substituents, and the numbers of carbons of the substituents are not included in the number of carbons in the divalent heterocyclic group. Substituents include halogen atoms and saturated or unsaturated hydrocarbon, aryl, alkoxy, aryloxy, monovalent heterocyclic, amino, nitro and cyano groups.

Also, either or both Z² and Z²' are preferably groups represented by formula (xii). Specifically, when m and o are both integers of 1 or greater, either or both Z² and Z^{2'} are preferably groups represented by formula (xii). When m is 0, Z^{2'} is preferably a group represented by formula (xii), and when o is 0, Z² is preferably a group represented by formula (xii).

In the branched compound of this embodiment, the core portion may be any organic group with value x having a structure in which the side chain portions and end portions can conjugate, examples of which include aromatic hydrocarbons with value x, heterocyclic groups with value x, residues of arylamines with value x and their derivatives, and organic groups that are combinations of the foregoing (provided that x is an integer of 3 or greater and corresponds to the number of side chain portions, same hereunder).

An aromatic hydrocarbon group with value x is an atomic group remaining after removing x hydrogens from a benzene ring or a fused ring, and the number of carbons is preferably 6-60 and more preferably 6-20. Fused rings include naphthalene, anthracene, tetracene, pentacene, pyrene, perylene, rubrene and fluorene rings. Particularly preferred among these are atomic groups remaining after removing x or more hydrogen atoms from a benzene ring. The x aromatic hydrocarbon groups may be optionally substituted. The numbers of carbon atoms of the substituents are not included in the number of carbon atoms in the x or more aromatic hydrocarbon groups. Substituents include halogen atoms and saturated or unsaturated hydrocarbon, aryl, alkoxy, aryloxy, monovalent heterocyclic, amino, nitro and cyano groups.

A heterocyclic group of value x is an atomic group remaining after removing x hydrogens from a heterocyclic compound, and the number of carbons is preferably 3-60 and more preferably 3-20. Heterocyclic compounds include thiophene, thienothiophene, dithienothiophene, pyrrole, pyridine, pyrimidine, pyrazine, triazine, benzothiazole and benzothiadiazole. Particularly preferred are atomic groups remaining after removing x hydrogens from thiophene, pyridine, pyrimidine or triazine. The x heterocyclic groups may have substituents, in which case the numbers of carbon atoms of the substituents are not included in the numbers of carbon atoms of the x heterocyclic groups. Substituents include halogen atoms and saturated or unsaturated hydrocarbon, aryl, alkoxy, aryloxy, monovalent heterocyclic, amino, nitro and cyano groups.

A residue of an arylamine of value x or its derivative is an atomic group remaining after removing x hydrogens from a compound having one or more aryl groups substituting on an amine, or a derivative such as a compound comprising a plurality of such compounds bonded together. Examples of arylamines and their derivatives include diphenylamine, triphenylamine, N,N'-tetraphenyl-phenylenediamine and N,N'-tetraphenyl-biphenylenediamine, with triphenylamine being preferred.

In the branched compound of this embodiment, the core portion is preferably any group represented by formulas (I) to (V), and more preferably a group represented by formula (II).

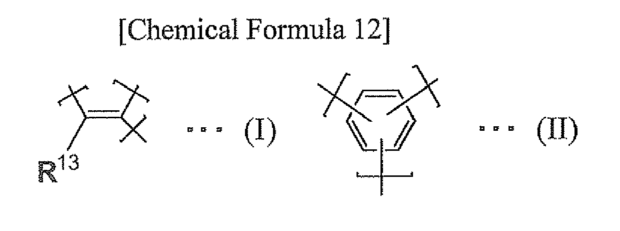

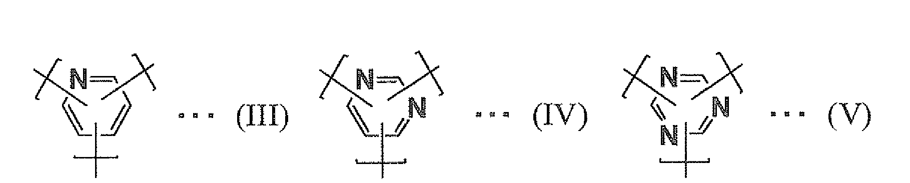

In the formula, R¹³ represents hydrogen, alkyl, aryl or cyano.

A branched compound comprising such a core portion has even more excellent conjugation, and can be utilized as an organic n-type semiconductor with an even more excellent electron transport property. In particular, when the core portion has such a structure and the side chain portions are groups represented by formula (3), conjugation extension occurs in a planar and three-dimensional manner throughout the entire molecule, facilitating interaction between molecules, and the electron transport property is vastly improved when the compound is used as an organic n-type semiconductor.

Preferred examples of substituents included in the structure described above will now be explained in detail. In the formula, the alkyl groups as R⁰-R¹³ are preferably C1-20 straight-chain, branched or cyclic alkyl groups, examples of which include methyl, ethyl, n-propyl, iso-propyl, n-butyl, iso-butyl, tert-butyl, sec-butyl, pentyl, hexyl, heptyl, octyl, nonyl, decyl, lauryl, cyclopropyl, cyclobutyl, cyclopentyl, cyclohexyl, cycloheptyl, cyclooctyl, cyclononyl and cyclododecyl. C1-12 alkyl groups are preferred, and pentyl, hexyl, octyl, decyl and cyclohexyl are more preferred.

Examples of alkoxy groups as R⁰-R¹² include alkoxy groups comprising the aforementioned alkyl groups in the structure.

Preferred examples of aryl groups as R⁰-R¹³ are C6-60 aryl groups, including phenyl and C¹-C¹² alkoxyphenyl (C¹-C¹² representing C1-12, same hereunder), C¹-C¹² alkylphenyl, 1-naphthyl and 2-naphthyl groups. Of these, C6-20 aryl groups are preferred, phenyl, C¹-C¹² alkoxyphenyl and C¹-C¹² alkylphenyl groups are more preferred, and phenyl is preferred.

Monovalent heterocyclic groups as R⁰-R¹² are preferably C4-60 monovalent heterocyclic groups, examples of which include thienyl, C¹-C¹² alkylthienyl, pyrrolyl, furyl, pyridyl and C¹-C¹² alkylpyridyl. C4-20 monovalent heterocyclic groups are preferred among these, and thienyl, C¹-C¹² alkylthienyl, pyridyl and C¹-C¹² alkylpyridyl are more preferred.

The branched compound of this embodiment will now be explained in detail. As mentioned above, the branched compound of this embodiment comprises a core portion, at least 3 side chain portions bonded to the core portion, and an end portion bonded to each of the side chain portions, wherein the side chain portions and the end portions bonded to the side chain portions are conjugated with the core portion. The side chain portions are composed of a plurality of linked conjugated units, and they preferably include at least one divalent heterocyclic group as a conjugated unit, being groups represented by formula (3). Since the branched compound preferably has an electron-withdrawing group in the end portion from the viewpoint of improving the electron transport property, at least one end portion may be a group represented by formula (1), and multiple end portions may be the same or different. From the viewpoint of facilitating production and interaction between molecules, multiple end groups are preferably the same.

Examples of branched compounds include branched compounds represented by formula (a) or (b).

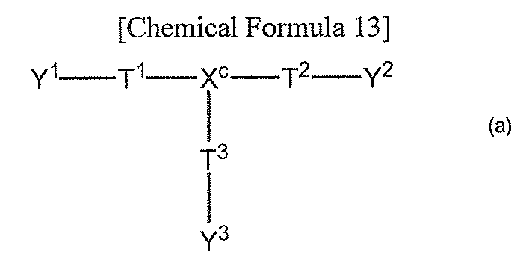

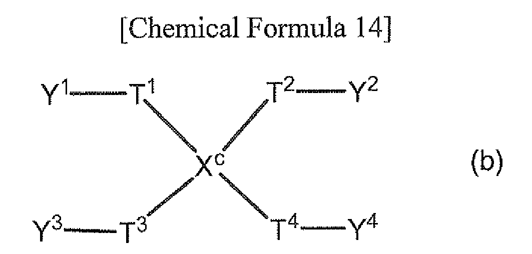

Here, X^{c} represents the core portion, T^{L} (L is an integer of 1-4) represents a side chain portion, and Y^{L} (L is an integer of 1-4) represents an end portion. T¹-T⁴ may be the same or different, and from the viewpoint of facilitating production they are preferably the same. Also, Y¹-Y⁴ may be the same or different, and from the viewpoint of facilitating production they are preferably the same.

The branched compound of this embodiment is more preferably a compound represented by formula (c), (d) or (e), from the viewpoint of further increasing the electron transport property and obtaining excellent stability.

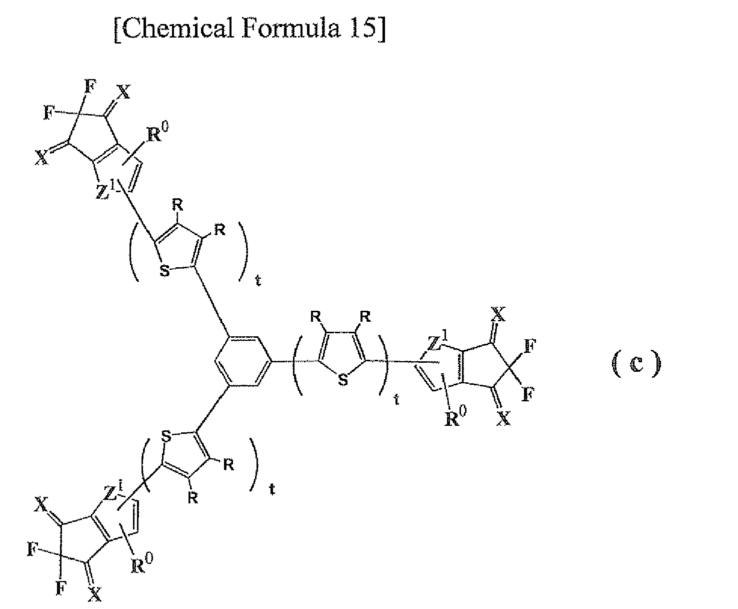

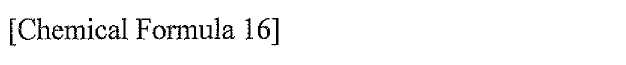

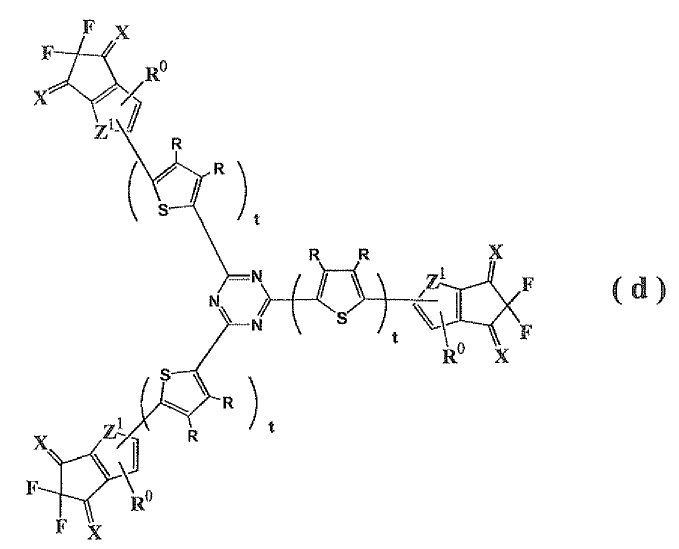

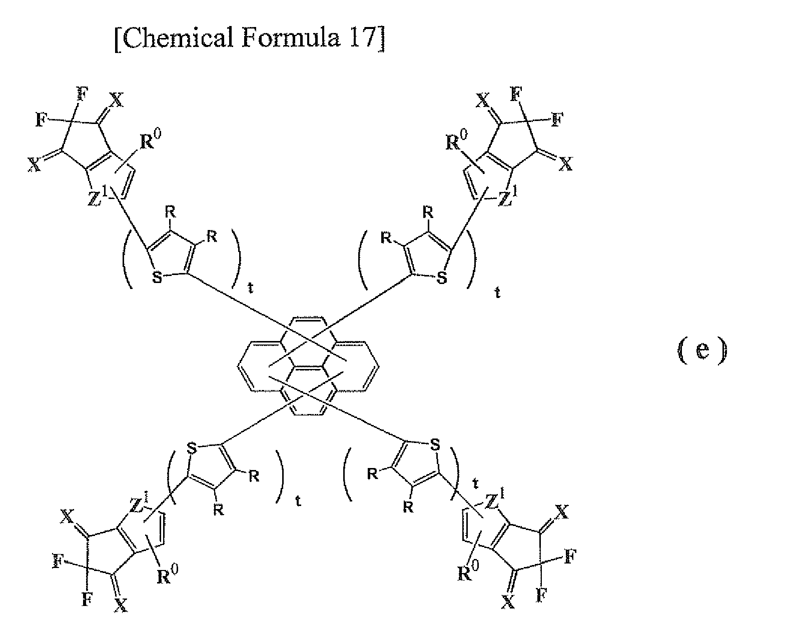

In formulas (c), (d) and (e), Z¹, X and R⁰ are the same as defined above, and multiple Z¹, X and R⁰ groups may be the same or different. R represents hydrogen or an alkyl group, and multiple R groups may be the same or different. Preferably, at least one of the substituents R on a plurality of linked thiophene rings is not hydrogen. The letter t represents an integer of 2-6. When multiple t groups are present, they may be the same or different.

The branched compound of this embodiment has a reduction potential based on ferrocene, as determined by electrochemical measurement (cyclic voltammetry), of preferably -2.0 V to +0.5 V and more preferably -1.8 V to +0.2 V. If the reduction potential is within this numerical range, the branched compound will be sufficiently suitable as an n-type semiconductor with a more excellent electron transport property. The reduction potential can be measured by the following method, for example.

For measurement of the reduction potential, an organic solvent is prepared containing about 0.1 mol/L tetrabutylammonium perchlorate and tetrabutylammonium hexafluorophosphate, as supporting electrolytes, and the material to be measured is dissolved therein to about 0.1-2 mM. The oxygen is removed from the obtained solution by a method such as dry nitrogen bubbling, vacuum deaeration or ultrasonic irradiation. Next, a platinum electrode or glassy carbon electrode is used as the work electrode with a platinum electrode as the counter electrode, for electrolytic reduction from an electrically neutral state at a sweep rate of 100 mV/sec. The potential of the first peak value detected during electrolytic reduction is compared with the oxidation-reduction potential of a reference material such as ferrocene, to obtain the oxidation (or reduction) potential for the material being measured. The value of the oxidation (or reduction) potential obtained in this manner, converted based on ferrocene, may be used as the reduction potential.

A method for producing a branched compound for this embodiment will now be explained. The branched compound may be produced by reacting compounds represented by formulas (IX) to (XIV), for example, as starting materials.

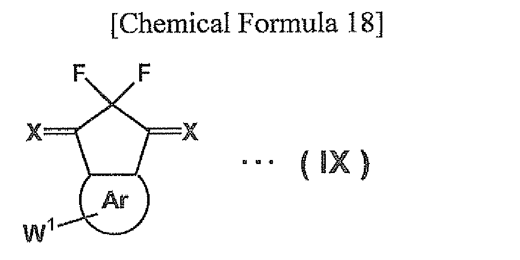

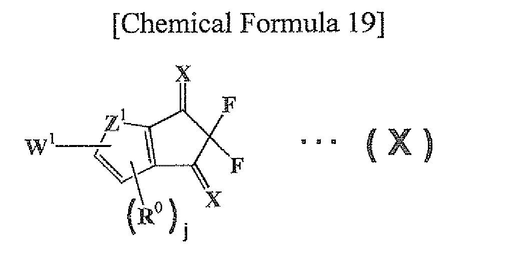

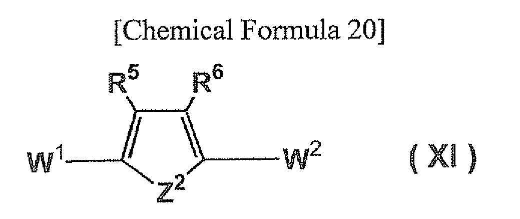

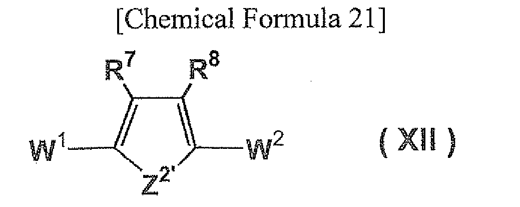

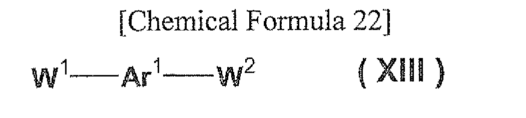

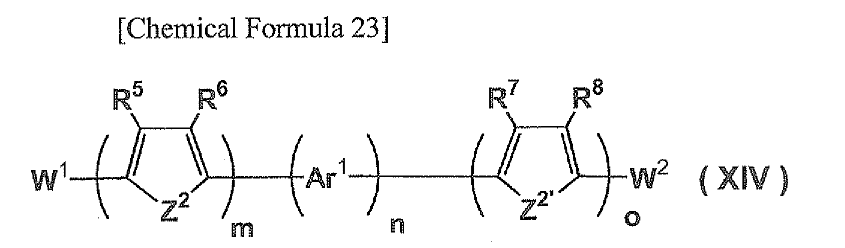

In formulas (IX) to (XIV), Ar, Ar¹, X, Z¹, Z², Z²', R⁰, R⁵-R⁸, m, n, o and j have the same definitions as above. W¹ and W² are the same or different, and each represents a halogen atom, alkyl sulfonate, aryl sulfonate, arylalkyl sulfonate, boric acid ester residue, sulfoniummethyl, phosphoniummethyl, phosphonatemethyl, monohalogenated methyl group, boric acid residue (-B(OH)₂), formyl, trialkylstannyl or vinyl group. Boric acid ester residues include dimethylboric acid, diisopropylboric acid, 1,3,2-dioxaborolane, 4,4,5,5-tetraethyl-1,3,2-dioxaborolane and 1,3,2-dioxaborolane.

From the viewpoint of facilitating synthesis and reaction of the compounds represented by formulas (IX) to (XIV), W¹ and W² are preferably the same or different groups from among halogen atoms, alkyl sulfonate, aryl sulfonate, arylalkyl sulfonate, boric acid ester residue, boric acid residue and trialkylstannyl groups. The groups represented by W¹ or W² are polymerization reactive groups that can create bonds by appropriate reaction.

When the starting material is a compound represented by formula (IX) or (X) wherein X is an oxygen atom, reaction will sometimes be hampered by the powerful electron-withdrawing property. In such cases, after reaction has been conducted using starting materials that are compounds represented by formula (IX') or (X') in which the carbonyl groups have been converted to alkylenedioxy groups, the alkylenedioxy groups of the compounds may be converted to carbonyl groups at an appropriate stage. In formulas (IX') and (X'), Ar, Z¹, R⁰ and W¹ have the same definitions as above.

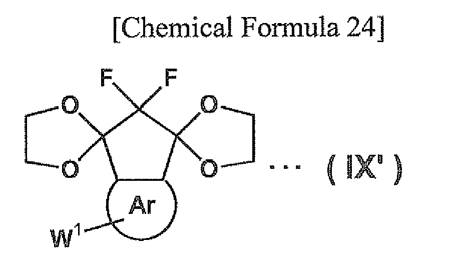

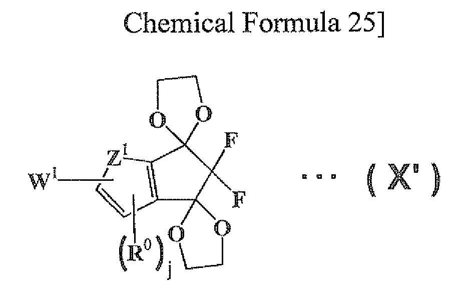

A method for producing a branched compound using such starting materials will now be described in detail.

Compounds represented by formula (IX) and preferably formula (X) are suitable examples of starting materials for the end portions, and compounds represented by formula (XI), (XII) or (XIII) and preferably formula (XIV), are suitable examples of starting materials for the side chain portions. Starting materials for the core portion include those wherein the bonding site with the side chain portion in the preferred structure of the core portion described above has been replaced with a group represented by W¹ or W². Using these starting materials, it is possible to obtain a branched compound by bonding together the starting materials by reaction between the groups represented by W¹ or W². The starting material compounds may be reacted in order while forming appropriate intermediate compounds, depending on the structure of the target branched compound.

The reaction for bonding between W¹ groups, between W² groups or between W¹ and W² groups may be a method using Suzuki coupling reaction, a method using Grignard reaction, a method using Stille reaction or a method using dehalogenation reaction.

Methods using Suzuki coupling reaction and methods using Stille reaction are preferred from the viewpoint of availability of the starting materials and convenience of the reaction procedure.

The catalyst used for Suzuki coupling reaction may be palladium [tetrakis(triphenylphosphine)] or palladium acetate, with addition of at least one equivalent and preferably 1-10 equivalents of an inorganic base such as potassium carbonate, sodium carbonate or barium hydroxide, an organic base such as triethylamine or an inorganic salt such as cesium fluoride, with respect to the monomer. In this case, the reaction may be carried out in a two-phase system, with the inorganic salt in aqueous solution. Examples of solvents include N,N-dimethylformamide, toluene, dimethoxyethane, tetrahydrofuran and the like. The reaction temperature will depend on the solvent used but is preferably 50-160°C. The temperature may be increased to near the boiling point of the solvent for reflux. The reaction time will be between 1 hour and 200 hours. Suzuki coupling reaction is described in Chem. Rev. Vol. 95, p.2457(1995).

For Stille reaction, a catalyst such as palladium [tetrakis(triphenylphosphine)] or palladium acetate may be used, and the reaction may be conducted using an organic tin compound as monomer. Examples of solvents include N,N-dimethylformamide, toluene, dimethoxyethane, tetrahydrofuran and the like. The reaction temperature will depend on the solvent used but is preferably 50-160°C. The temperature may be increased to near the boiling point of the solvent for reflux. The reaction time will be between 1 hour and 200 hours.

Examples for the polymerization reactive groups W¹ and W² include halogen, alkyl sulfonate, aryl sulfonate, arylalkyl sulfonate, boric acid ester residue, sulfoniummethyl, phosphoniummethyl, phosphonatemethyl, monohalogenated methyl, boric acid residue, formyl, alkylstannyl and vinyl groups, and these may be used in appropriate combinations depending on the reaction in which they are used. Examples of boric acid ester residues include groups represented by the following formulas.

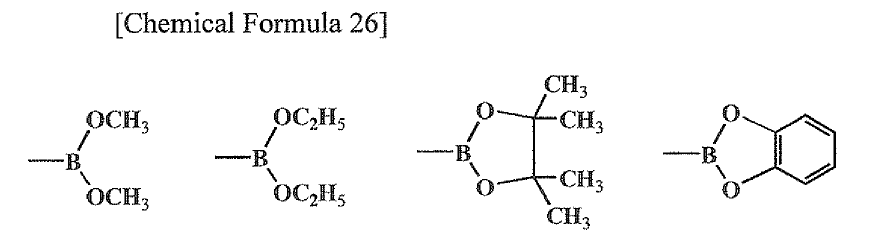

Preferred combinations of active functional groups W¹ and W² are combinations of halogen atoms and boric acid ester residues or boric acid residues, for methods using Suzuki coupling reaction, and combinations of halogen atoms and alkylstannyl groups, for methods using Stille reactions.

Any desired sites may be protected with protecting groups during the reaction. Protecting groups may be selected as suitable groups depending on the site to be protected and the reaction employed, and examples of preferred protecting groups are mentioned in "Protective Groups in Organic Synthesis, 3rd ed. T.W. Greene and P.G.M. Wuts, 1999 John Willey & Sons, Inc.". When the site to be protected is an alkyne, examples include trialkylsilyl groups such as trimethylsilyl, triethylsilyl and t-butyldimethylsilyl, aryldialkylsilyl groups such as biphenyldimethylsilyl, and 2-hydroxypropyl groups, with trimethylsilyl being preferred.

The starting materials (monomers) to be reacted are dissolved in an organic solvent, or an alkali or an appropriate catalyst is used, and reaction is conducted at a temperature above the melting point and below the boiling point of the organic solvent.

The organic solvent used will differ depending on the compounds and reaction employed, but in order to limit secondary reactions it is generally preferred to be one that accomplishes sufficient deoxygenation treatment and promotes the reaction in an inert atmosphere. Similarly, dehydrating treatment is also preferably carried out (although dehydrating treatment is not necessary in cases of reaction conducted in a two-phase system with water, such as in Suzuki coupling reaction).

An appropriate alkali or an appropriate catalyst may be added during production of the branched compound of this embodiment, and they may be selected according to the reaction employed. The alkali or catalyst used is preferably one that thoroughly dissolves in the solvent used for the reaction.

When the branched compound of this embodiment is to be used as a material for an organic thin-film element, its purity can affect the element characteristics. It is therefore preferred to use the starting materials in the reaction after purification by a method such as distillation, sublimation purification or recrystallization, and the synthesis is also preferably followed by purifying treatment, such as sublimation purification, recrystallization, reprecipitating purification or chromatographic separation.

Examples of solvents to be used for the reaction include saturated hydrocarbons such as pentane, hexane, heptane, octane and cyclohexane, unsaturated hydrocarbons such as benzene, toluene, ethylbenzene and xylene, halogenated saturated hydrocarbons such as carbon tetrachloride, chloroform, dichloromethane, chlorobutane, bromobutane, chloropentane, bromopentane, chlorohexane, bromohexane, chlorocyclohexane and bromocyclohexane, halogenated unsaturated hydrocarbons such as chlorobenzene, dichlorobenzene and trichlorobenzene, alcohols such as methanol, ethanol, propanol, isopropanol, butanol and t-butyl alcohol, carboxylic acids such as formic acid, acetic acid and propionic acid, ethers such as dimethyl ether, diethyl ether, methyl-t-butyl ether, tetrahydrofuran, tetrahydropyran and dioxane, and inorganic acids such as hydrochloric acid, hydrobromic acid, hydrofluoric acid, sulfuric acid and nitric acid. These may be used as simple solvents or as mixed solvents.

The reaction may be followed by ordinary post-treatment such as quenching with water, subsequent extraction with an organic solvent and distillation of the solvent to obtain a product. Isolation and purification of the product can be carried out by chromatographic fractionation or recrystallization.

### (Organic thin-film)

An organic thin-flm according to this embodiment will now be explained. The organic thin-film of this embodiment comprises the branched compound described above.

The thickness of the organic thin-film will usually be 1 nm-100 µm, preferably 2 nm-1000 nm, even more preferably 5 nm-500 nm and most preferably 20 nm-200 nm.

The organic thin-film may be one comprising only one of the aforementioned branched compounds, or it may include two or more of such branched compounds. In order to enhance the electron transport property and hole transport property of the organic thin-film, an electron transport material and a hole transport material may be used in admixture, in addition to the branched compound.

Any known hole transport material may be used, examples of which include pyrazoline derivatives, arylamine derivatives, stilbene derivatives, triaryldiamine derivatives, oligothiophene and its derivatives, polyvinylcarbazole and its derivatives, polysilane and its derivatives, polysiloxane derivatives with aromatic amines on the side chains or main chain, polyaniline and its derivatives, polythiophene and its derivatives, polypyrrole and its derivatives, polyarylenevinylene and its derivatives, and polythienylenevinylene and its derivatives. Any known electron transport material may be used, examples of which include oxadiazole derivatives, quinodimethane and its derivatives, benzoquinone and its derivatives, naphthoquinone and its derivatives, anthraquinone and its derivatives, tetracyanoanthraquinodimethane and its derivatives, fluorenone derivatives, diphenyldicyanoethylene and its derivatives, diphenoquinone derivatives, metal complexes of 8-hydroxyquinoline and its derivatives, polyquinoline and its derivatives, polyquinoxaline and its derivatives, polyfluorene and its derivatives, and C₆₀ or other fullerenes and their derivatives.

An organic thin-film according to this embodiment may also contain a charge generating material for generation of an electrical charge upon absorption of light in the organic thin-film. Any publicly known charge generating material may be used, and examples include azo compounds and their derivatives, diazo compounds and their derivatives, ametallic phthalocyanine compounds and their derivatives, metallic phthalocyanine compounds and their derivatives, perylene compounds and their derivatives, polycyclic quinone-based compounds and their derivatives, squarylium compounds and their derivatives, azulenium compounds and their derivatives, thiapyrylium compounds and their derivatives, and C₆₀ or other fullerenes and their derivatives.

The organic thin-film of this embodiment may also contain other materials necessary for exhibiting various functions. Examples of such other materials include sensitizing agents to enhance the function of generating charge by light absorption, stabilizers to increase stability, and UV absorbers for absorption of UV light.

The organic thin-film of this embodiment may also contain high molecular compound materials as macromolecular binders in addition to the branched compound, in order to improve the mechanical properties. As macromolecular binders there are preferably used ones that produce minimal interference with the electron transport or hole transport property, and ones with weak absorption for visible light.

Examples of such high molecular binders include poly(N-vinylcarbazole), polyaniline and its derivatives, polythiophene and its derivatives, poly(p-phenylenevinylene) and its derivatives, poly(2,5-thienylenevinylene) and its derivatives, polycarbonates, polyacrylates, polymethyl acrylates, polymethyl methacrylates, polystyrenes, polyvinyl chlorides, polysiloxanes and the like.

There are no particular restrictions on the method for producing an organic thin-film according to this embodiment, and there may be employed a method of film formation using a solution comprising the branched compound and, as necessary, an electron transport or hole transport material and a high molecular binder in admixture therewith. The branched compound can be formed into a thin-film by a vacuum vapor deposition method.

The solvent used for film formation from a solution may be any one that dissolves the branched compound and the electron transport material or hole transport material and high molecular binders combined therewith.

Examples of solvents to be used for formation of the organic thin-film of this embodiment from a solution include unsaturated hydrocarbon-based solvents such as toluene, xylene, mesitylene, tetralin, decalin, bicyclohexyl, n-butylbenzene, sec-butylbenzene and tert-butylbenzene, halogenated saturated hydrocarbon-based solvents such as carbon tetrachloride, chloroform, dichloromethane, dichloroethane, chlorobutane, bromobutane, chloropentane, bromopentane, chlorohexane, bromohexane, chlorocyclohexane and bromocyclohexane, halogenated unsaturated hydrocarbon-based solvents such as chlorobenzene, dichlorobenzene and trichlorobenzene, and ether-based solvents such as tetrahydrofuran and tetrahydropyran. The branched compound may be dissolved in such solvents to at least 0.1 wt%, although this will differ depending on the structure and molecular weight of the polymer.

Formation of a film from a solution may be accomplished using a coating method such as spin coating, casting, microgravure coating, gravure coating, bar coating, roll coating, wire bar coating, dip coating, spray coating, screen printing, flexographic printing, offset printing , ink jet printing, dispenser printing or the like, with spin coating, flexographic printing, ink jet printing and dispenser printing methods being preferred.

The organic thin-film of this embodiment is preferably one that has been subjected to annealing treatment after film formation. Annealing treatment improves the quality of the organic thin-film, by promoting interaction between the branched compounds, for example, and increases the electron mobility or hole mobility. The treatment temperature for annealing is preferably a temperature between 50°C and near the glass transition temperature (Tg) of the branched compound, and more preferably a temperature between (Tg-30°C) and Tg. The annealing treatment time is preferably from 1 minute to 10 hours and more preferably from 10 minutes to 1 hour. The atmosphere for annealing treatment is preferably a vacuum or an inert gas atmosphere.

Since the organic thin-film of this embodiment has an electron transport property or hole transport property, the transport of electrons or holes introduced from the electrode or charge generated by photoabsorption can be controlled for use in various organic thin-film elements such as organic thin-film transistors, organic thin-film light-emitting transistors, organic solar cells and optical sensors, as described below. Examples of preferred organic thin-film elements will now be described.

### (Organic thin-film transistor)

An organic thin-film transistor according to a preferred embodiment will be explained first. The organic thin-film transistor may have a structure comprising a source electrode and drain electrode, an active layer (preferably an organic thin-film layer, same hereunder) containing a branched compound of the invention which acts as a current channel between them, and a gate electrode that controls the level of current flowing through the current channel, and the transistor may be a field-effect type or static induction type, for example.

An organic thin-film field-effect transistor may have a structure comprising a source electrode and drain electrode, an active layer containing a preferred branched compound mentioned above which acts as a current channel between them, a gate electrode that controls the level of current flowing through the current channel, and an insulating layer situated between the active layer and the gate electrode. Preferably, the source electrode and drain electrode are provided in contact with the active layer containing the branched compound, and the gate electrode is provided sandwiching the insulating layer which is also in contact with the active layer.

A static induction-type organic thin-film transistor has a structure comprising a source electrode and drain electrode, an active layer containing a branched compound which acts as a current channel between them and a gate electrode that controls the level of current flowing through the current channel, preferably with the gate electrode in the active layer. Most preferably, the source electrode, the drain electrode and the gate electrode formed in the active layer are provided in contact with the active layer containing the branched compound. The structure of the gate electrode may be any one that forms a current channel for flow from the source electrode to the drain electrode, and that allows the level of current flowing through the current channel to be controlled by the voltage applied to the gate electrode; an example of such a structure is a combshaped electrode.

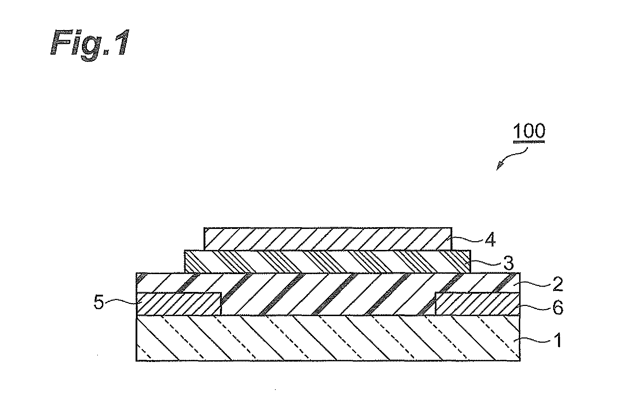

Fig. 1 is a schematic cross-sectional view of an organic thin-film transistor (organic thin-film field-effect transistor) according to a first embodiment. The organic thin-film transistor 100 shown in Fig. 1 comprises a substrate 1, a source electrode 5 and drain electrode 6 formed at a fixed spacing on the substrate 1, an active layer 2 formed on the substrate 1 covering the source electrode 5 and drain electrode 6, an insulating layer 3 formed on the active layer 2, and a gate electrode 4 formed on the insulating layer 3 covering the region of the insulating layer 3 between the source electrode 5 and drain electrode 6.

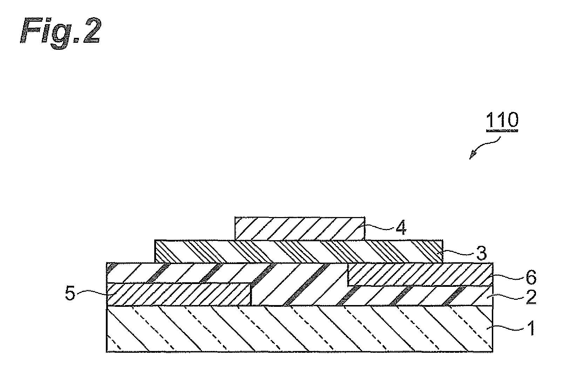

Fig. 2 is a schematic cross-sectional view of an organic thin-film transistor (organic thin-film field-effect transistor) according to a second embodiment. The organic thin-film transistor 110 shown in Fig. 2 comprises a substrate 1, a source electrode 5 formed on the substrate 1, an active layer 2 formed on the substrate 1 covering the source electrode 5, a drain electrode 6 formed on the active layer 2 at a prescribed spacing from the source electrode 5, an insulating layer 3 formed on the active layer 2 and drain electrode 6, and a gate electrode 4 formed on the insulating layer 3 covering the region of the insulating layer 3 between the source electrode 5 and drain electrode 6.

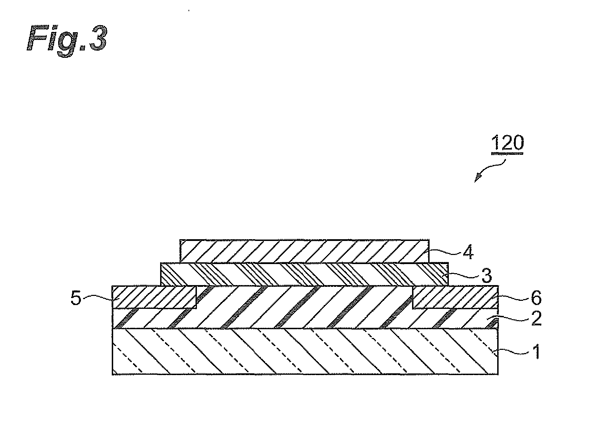

Fig. 3 is a schematic cross-sectional view of an organic thin-film transistor (organic thin-flm field-effect transistor) according to a third embodiment. The organic thin-film transistor 120 shown in Fig. 3 comprises a substrate 1, an active layer 2 formed on the substrate 1, a source electrode 5 and drain electrode 6 formed at a prescribed spacing on the active layer 2, an insulating layer 3 formed on the active layer 2 covering the source electrode 5 and drain electrode 6, and a gate electrode 4 formed on the insulating layer 3, covering a portion of the region of the insulating layer 3 under which the source electrode 5 is formed and a portion of the region of the insulating layer 3 under which the drain electrode 6 is formed.

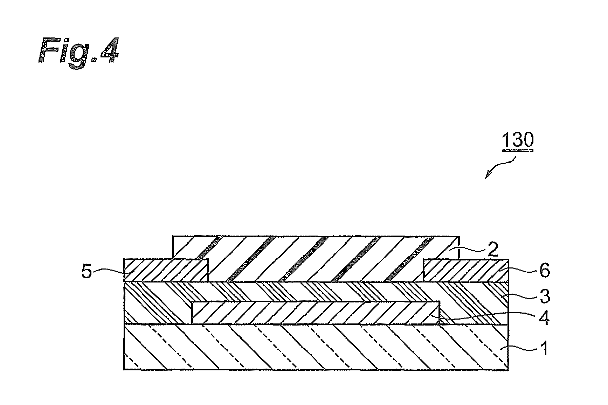

Fig. 4 is a schematic cross-sectional view of an organic thin-film transistor (organic thin-film field-effect transistor) according to a fourth embodiment. The organic thin-film transistor 130 shown in Fig. 4 comprises a substrate 1, a gate electrode 4 formed on the substrate 1, an insulating layer 3 formed on the substrate 1 covering the gate electrode 4, a source electrode 5 and drain electrode 6 formed at a prescribed spacing on the insulating layer 3 covering portions of the region of the insulating layer 3 under which the gate electrode 4 is formed, and an active layer 2 formed on the insulating layer 3 and covering portions of the source electrode 5 and drain electrode 6.

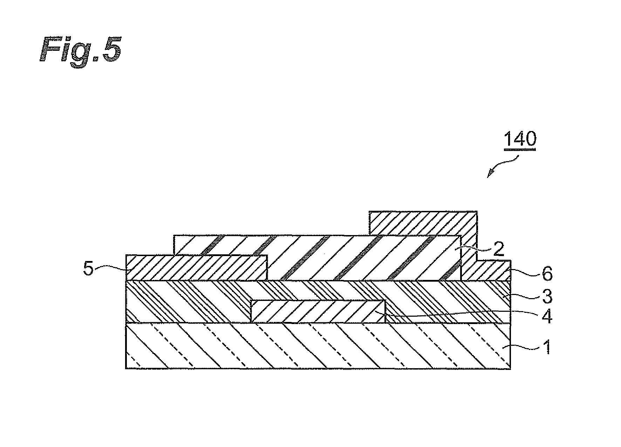

Fig. 5 is a schematic cross-sectional view of an organic thin-film transistor (organic thin-film field-effect transistor) according to a fifth embodiment. The organic thin-film transistor 140 shown in Fig. 5 comprises a substrate 1, a gate electrode 4 formed on the substrate 1, an insulating layer 3 formed on the substrate 1 covering the gate electrode 4, a source electrode 5 formed on the insulating layer 3 covering a portion of the region of the insulating layer 3 under which the gate electrode 4 is formed, an active layer 2 formed on the insulating layer 3 covering a portion of the source electrode 5, and a drain electrode 6 formed on the insulating layer 3 at a prescribed spacing from the source electrode 5 and covering a portion of the region of the active layer 2.

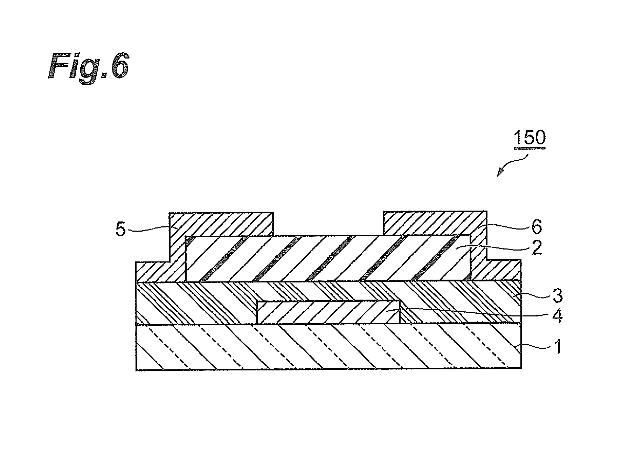

Fig. 6 is a schematic cross-sectional view of an organic thin-film transistor (organic thin-film field-effect transistor) according to a sixth embodiment. The organic thin-film transistor 150 shown in Fig. 6 comprises a substrate 1, a gate electrode 4 formed on the substrate 1, an insulating layer 3 formed on the substrate 1 covering the gate electrode 4, an active layer 2 formed covering the region of the insulating layer 3 under which the gate electrode 4 is formed, a source electrode 5 formed on the insulating layer 3 covering a portion of the region of the active layer 2, and a drain electrode 6 formed on the insulating layer 3 at a prescribed spacing from the source electrode 5 and covering a portion of the region of the active layer 2.

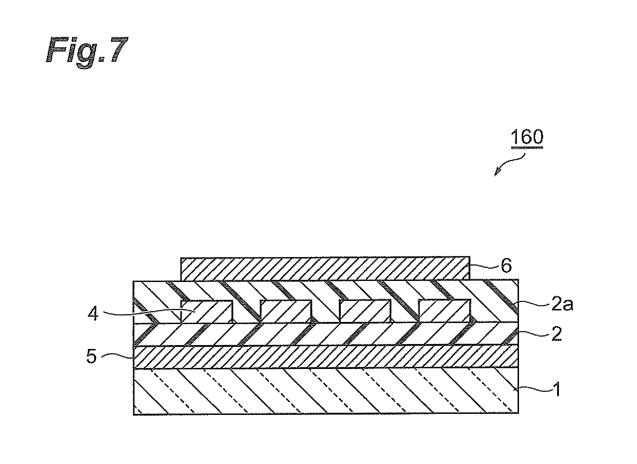

Fig. 7 is a schematic cross-sectional view of an organic thin-film transistor (static induction-type organic thin-film transistor) according to a seventh embodiment. The organic thin-film transistor 160 shown in Fig. 7 comprises a substrate 1, a source electrode 5 formed on the substrate 1, an active layer 2 formed on the source electrode 5, a plurality of gate electrodes 4 formed at prescribed spacings on the active layer 2, an active layer 2a formed on the active layer 2 covering all of the gate electrodes 4, (the material composing the active layer 2a may be the same as or different from that of the active layer 2), and a drain electrode 6 formed on the active layer 2a.

In the organic thin-film transistors of the first to seventh embodiments, the active layer 2 and/or the active layer 2a contains a preferred branched compound described above and forms a current channel between the source electrode 5 and drain electrode 6. The gate electrode 4 controls the level of current flowing through the current channel of the active layer 2 and/or active layer 2a by application of voltage.

This type of organic thin-film field-effect transistor can be manufactured by a publicly known process, such as the process described in Japanese Unexamined Patent Application Publication HEI No. 5-110069, for example. A static induction-type organic thin-film transistor can be manufactured by a publicly known process, such as the process described in Japanese Unexamined Patent Application Publication No. 2004-006476, for example.

The substrate 1 may be any one that does not impair the characteristics of the organic thin-film transistor, and a glass panel, flexible film substrate or plastic panel may be used.

Although organic solvent-soluble conjugated compounds are highly advantageous in terms of production and preferred for forming the active layer 2, the conjugated compounds mentioned above have excellent solubility and thus allow formation of an organic thin-film comprising the active layer 2 by the method for producing an organic thin-film described above.

The insulating layer 3 in contact with the active layer 2 may be any material with high electrical insulating properties, and any publicly known one may be used. As examples there may be mentioned SiOx, SiNx, Ta₂O₅, polyimide, polyvinyl alcohol, polyvinylphenol and organic glass. From the viewpoint of low voltage, a material with high permittivity is preferred.

When the active layer 2 is formed on the insulating layer 3, the active layer 2 may be formed after surface modification by treatment of the surface of the insulating layer 3 with a surface treatment agent such as a silane coupling agent in order to improve the interfacial properties between the insulating layer 3 and active layer 2. Surface treatment agents include long-chain alkylchlorosilanes, long-chain alkylalkoxysilanes, fluorinated alkylchlorosilanes, fluorinated alkylalkoxysilanes and silylamine compounds such as hexamethyldisilazane. Before treatment with the surface treatment agent, the insulating layer surface may be pre-treated by ozone UV or O₂ plasma.

After the organic thin-film transistor has been fabricated, a protecting film is preferably formed on the organic thin-film transistor to protect the element. This will help prevent reduction in the characteristics of the organic thin-film transistor when the organic thin-film transistor has been blocked from air. A protecting film can also minimize adverse effects when an operating display device is formed on the organic thin-film transistor.

The method of forming the protecting film may involve covering with a UV curing resin, thermosetting resin, inorganic SiONx film or the like. For effective shielding from air, the steps after fabrication of the organic thin-film transistor and before formation of the protecting film are preferably carried out without exposure to air (for example, in a dry nitrogen atmosphere or in a vacuum).

The organic thin-film transistor of the invention may be used as an organic thin-film light-emitting transistor, if the active layer employs the aforementioned branched compounds that function as bipolar organic semiconductors.

### (Organic solar cell)

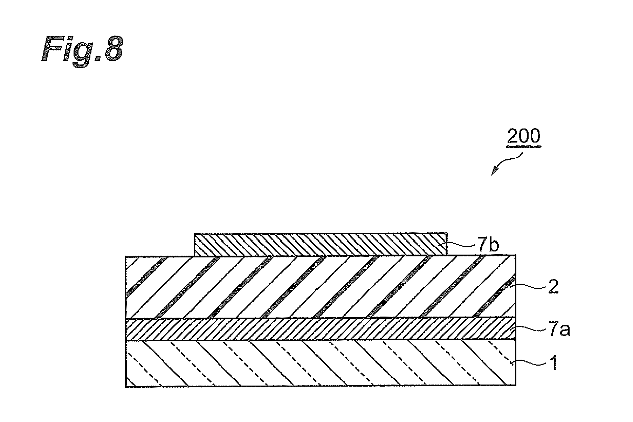

Application of an organic thin-film of the invention in a solar cell (organic solar cell) will now be explained. Fig. 8 is a schematic cross-sectional view of a solar cell according to an embodiment of the invention. The solar cell 200 shown in Fig. 8 comprises a substrate 1, a first electrode 7a formed on the substrate 1, an active layer 2 made of an organic thin-film that contains a preferred branched compound mentioned above formed on the first electrode 7a, and a second electrode 7b formed on the active layer 2.

In the solar cell of this embodiment, a transparent or semi-transparent electrode is used for either or both the first electrode 7a and second electrode 7b. As electrode materials there may be used metals such as aluminum, gold, silver, copper, alkali metal and alkaline earth metals or their semi-transparent films, or transparent conductive films. In order to obtain high open voltage, it is preferred to select the electrodes so as to produce a large work function difference. Carrier generators, sensitizing agents and the like may also be added in order to increase photosensitivity in the active layer 2 (organic thin-film). The substrate 1 may be a silicon substrate, glass panel, plastic panel or the like.

### (Optical sensor)

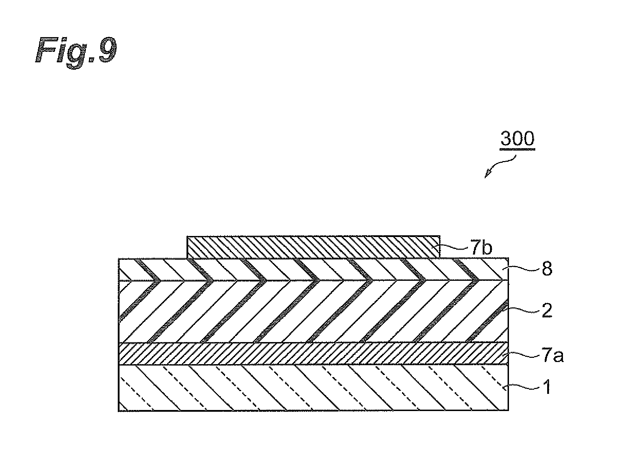

Application of an organic thin-film of the invention in an optical sensor will now be explained. Fig. 9 is a schematic cross-sectional view of an optical sensor according to a first embodiment. The optical sensor 300 shown in Fig. 9 comprises a substrate 1, a first electrode 7a formed on the substrate 1, an active layer 2 made of an organic thin-film comprising a preferred branched compound mentioned above formed on the first electrode 7a, a charge generation layer 8 formed on the active layer 2, and a second electrode 7b formed on the charge generation layer 8.

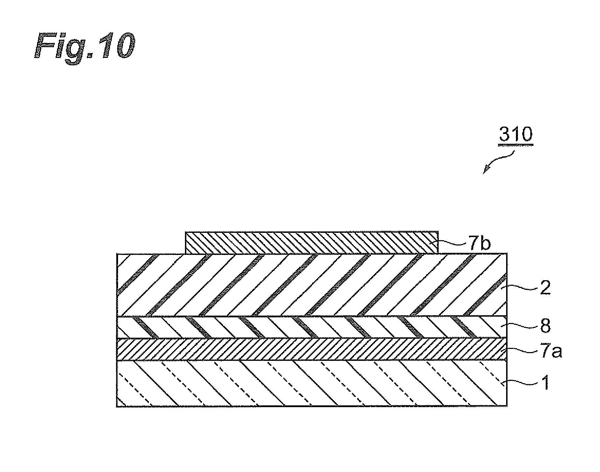

Fig. 10 is a schematic cross-sectional view of an optical sensor according to a second embodiment. The optical sensor 310 shown in Fig. 10 comprises a substrate 1, a first electrode 7a formed on the substrate 1, a charge generation layer 8 formed on the first electrode 7a, an active layer 2 made of an organic thin-film comprising a branched compound of the invention, formed on the charge generation layer 8, and a second electrode 7b formed on the active layer 2.

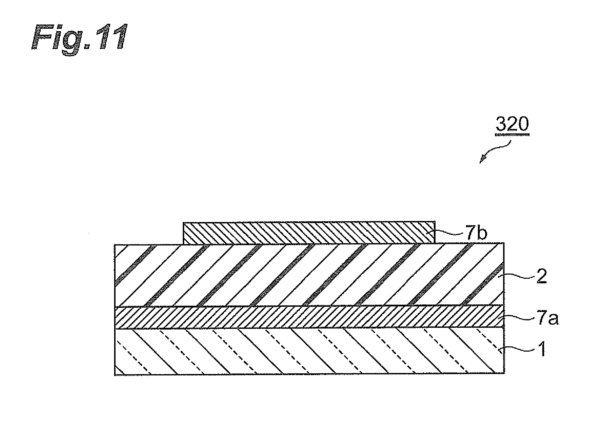

Fig. 11 is a schematic cross-sectional view of an optical sensor according to a third embodiment. The optical sensor 320 shown in Fig. 11 comprises a substrate 1, a first electrode 7a formed on the substrate 1, an active layer 2 made of an organic thin-film that comprises a branched compound of the invention, formed on the first electrode 7a, and a second electrode 7b formed on the active layer 2.

In the optical sensors of the first to third embodiments, a transparent or semi-transparent electrode is used for either or both the first electrode 7a and second electrode 7b. The charge generation layer 8 is a layer that generates an electrical charge upon absorption of light. As electrode materials there may be used metals such as aluminum, gold, silver, copper, alkali metal and alkaline earth metals or their semi-transparent films, or transparent conductive films. Carrier generators, sensitizing agents and the like may also be added in order to increase photosensitivity in the active layer 2 (organic thin-film). The substrate 1 may be a silicon substrate, glass panel, plastic panel or the like.

### Examples

The present invention will now be explained in greater detail based on examples and comparative examples, with the understanding that the invention is in no way limited to the examples.

### (Measuring conditions)

The nuclear magnetic resonance (NMR) spectra were measured using a JMN-270 (270 MHz for ¹H measurement) or a JMNLA-600 (150 MHz for ¹³C measurement), both trade names of JEOL Corp. The chemical shifts are represented as parts per million (ppm). Tetramethylsilane (TMS) was used as the internal standard (0 ppm). The coupling constant (J) is represented in Hz, and the symbols s, d, t, m and br respectively represent singlet, doublet, triplet, quartet, multiplet and broad.

Mass spectrometry (MS) was conducted using a Voyager Linear DE-H MALDI-TOF MS (trade name) by PerSeptive Biosystems. The silica gel used for separation by column chromatography was Silicagel 60N (40-50 µm), trade name of Kanto Kagaku Co., Ltd. The alumina used was standardized Aluminium Oxide 90, trade name of Merck. All of the chemical substances were reagent grade and purchased from Wako Pure Chemical Industries, Ltd., Tokyo Kasei Kogyo Co., Ltd., Kanto Kagaku Co., Ltd., Nacalai Tesque, Inc. or Sigma Aldrich Japan, KK.

Cyclic voltammetry was performed using an apparatus by BAS, Inc., with a Pt electrode by BAS, Inc. as the work electrode, Pt wire as the counter electrode and Ag wire as the reference electrode. The sweep rate during measurement was 100 mV/sec, and the scanning potential range was -2.8 V to 1.6 V. The reduction potential and oxidation potential were measured after completely dissolving 1 × 10⁻³ mol/L of the compound and 0.1 mol/L of tetrabutylammonium hexafluorophosphate (TBAPF6) as a supporting electrolyte in a methylene chloride solvent.

### (Synthesis Example 1)

Following scheme 1 shown below, compound A represented by formula (23a) was used as starting material for synthesis of compound D represented by formula (25), as the starting material for the branched compound, via compound B represented by formula (23b) and compound C represented by formula (24). This will be explained in detail below.

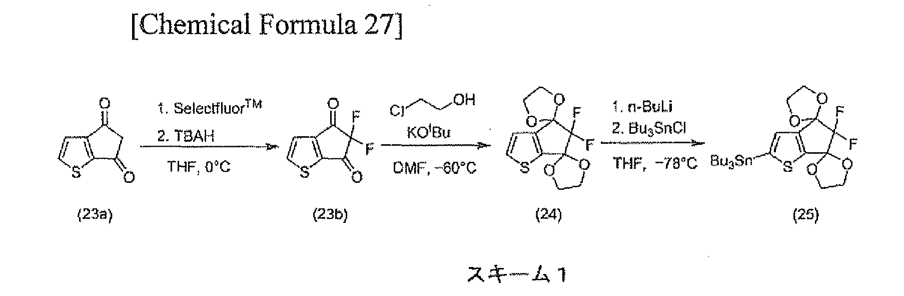

### Scheme 1

### <Synthesis of Compound B>

Compound A represented by formula (23 a) was synthesized by a method described in the literature (J. Chem. Soc. Perkin Trans, 1. Organic and Bio-Organic Chemistry 1992, 21, 2985-2988). Next, compound A (1.00 g, 6.58 mol) and the fluorinating agent Selectfluor^{™} (registered trademark) (5.60 g, 15.8 mol) were placed in a 300 mL three-necked flask and THF (65 mL) was added to dissolve them. Tetrabutylammonium hydroxide (TBAH) (10% methanol solution) (3.76 g, 14.5 mol) was then added and the mixture was stirred at 0°C for 12 hours. The solvent was distilled off under reduced pressure, and then water was added, the aqueous layer was extracted with ethyl acetate, and the organic layer was dried over magnesium sulfate and concentrated under reduced pressure. The obtained concentrate was purified by silica gel column chromatography (hexane/ethyl acetate = 3/1 (volume ratio)) to obtain compound B represented by formula (23b) (0.934 g, 75% yield) as a light yellow solid.

The evaluation results for the obtained compound B are as follows. mp 156-158°C; TLC R_{f} = 0.29 (2/1 = hexane/ethyl acetate (volume ratio)); ¹H-NMR (400 MHz, CDCl₃) δ 7.60 (d, 1H, J = 4.8 Hz), 8.28 (d, 1H, J = 4.8 Hz); MS (EI) m/z = 188 (M+)

### <Synthesis of Compound C>

Compound B (1.97 g, 10.48 mmol) was placed in a 200 mL three-necked flask, N,N'-dimethylformamide (DMF) (50 mL) was added to dissolve it, and then 2-chloromethanol (3.37 g, 41.91 mmol) was further added. Potassium tert-butoxide dissolved in DMF (50 mL) was then added dropwise thereto at -60°C. Upon completion of the dropwise addition, the mixture was stirred at room temperature for 4 hours, and water was added to suspend the reaction. The aqueous layer was extracted with ethyl acetate and rinsed with water, and then the organic layer was dried over magnesium sulfate, filtered and concentrated under reduced pressure. The obtained concentrate was purified by silica gel column chromatography (hexane/ethyl acetate = 3/1 (volume ratio)) to obtain compound C represented by formula (24) (1.58 g, 55% yield) as a white solid.

The evaluation results for the obtained compound C are as follows.
mp 117-122°C; TLC R_{f} = 0.34 (2/1 = hexane/ethyl acetate (volume ratio)); ¹H-NMR (400 MHz, CDCl₃) δ 4.26 (s, 8H), 7.02 (d, 1H, J = 4.8 Hz), 7.51 (d, 1H, J = 5.1 Hz); MS (EI) m/z = 276(M⁺)

### <Synthesis of Compound D>

Compound C (500 mg, 1.81 mmol) was placed in a 50 mL three-necked flask, and THF (18 mL) was added to dissolve it. Next, n-butyllithium (1.58 M hexane solution, 2.29 mL, 3.62 mmol) was added thereto at -78°C. After stirring for 0.5 hour, tributyltin chloride (1.09 mL, 3.98 mmol) was added and the temperature was slowly raised to room temperature. After 1 hour, water was added to suspend the reaction. The aqueous layer was extracted with ethyl acetate and rinsed with water, and then the organic layer was dried over magnesium sulfate, filtered and concentrated under reduced pressure. The obtained concentrate was purified by alumina-column chromatography (hexane/ethyl acetate = 10/1 (volume ratio)) to obtain compound D represented by formula (25) (1.02 g, 99% yield) as a colorless liquid.

The evaluation results for the obtained compound D are as follows.
TLC R_{f} = 0.30(hexane); ¹H-NMR (400 MHz, CDCl₃) δ 0.89 (t, 9H, J = 7.2 Hz), 1.08-1.13 (m, 6H), 1.24-1.38 (m, 6H), 1.49-1.60 (m, 6H), 4.23-4.28 (m, 8H), 7.03 (s, 1H); MS (EI) m/z = 566 (M⁺)

### (Synthesis Example 2)

Compound D obtained as described above was used for synthesis of compound F as an intermediate compound, via compound E.

### <Synthesis of Compound E>

After placing 2-bromo-3-hexylthiophene (600 mg, 2.43 mmol), compound D (1.51 g, 2.67 mmol) and tetrakis(triphenylphosphine)palladium(0) (281 mg, 0.243 mmol) in a heat-dried stoppered test tube, toluene (25 mL) was added to dissolve them. After stirring the mixture at 120°C for 12 hours, it was allowed to cool at room temperature. The solvent was distilled off under reduced pressure, and the obtained crude product was purified by silica gel column chromatography (hexane/ethyl acetate = 10/1 (volume ratio)) to obtain compound E represented by formula (26) (960 mg, 8 1 % yield) as a yellow liquid.

The evaluation results for the obtained compound E are as follows.
TLC R_{f} = 0.46(5/1 = hexane/ethyl acetate (volume ratio)); ¹H-NMR (400 MHz, CDCl₃) δ 0.89 (t, 3H, J = 3.6 Hz), 1.23-1.43 (m, 4H), 1.53-1.69 (m, 4H), 2.72 (t, 2H, J = 8.0 Hz), 4.27 (s, 8H), 6.94 (d, 1H, J = 5.4 Hz), 6.97 (s, 1H), 7.22 (d, 1H, J = 5.4 Hz); MS (EI) m/z442 (M⁺).

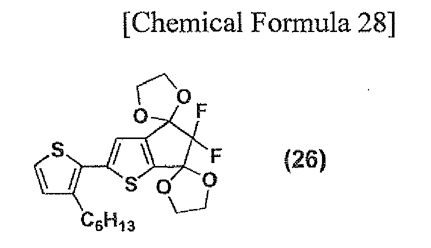

### <Synthesis of Compound F>

Compound E (200 mg, 0.452 mmol) was placed in a 50 mL two-necked flask, and was dissolved in THF (6 mL). Next, n-butyllithium (1.66 M hexane solution, 0.30 mL, 0.498 mmol) was added thereto at -78°C. After stirring for 1 hour, bromine (86 mg, 0.542 mmol) was added and the temperature was slowly raised to room temperature. After 0.5 hour, water was added to suspend the reaction. The aqueous layer was extracted with ethyl acetate and rinsed with saturated aqueous sodium thiosulfate and then with brine, and the organic layer was dried over magnesium sulfate. The crude product obtained by distilling off the solvent under reduced pressure was transferred to a 50 mL volumetric flask and dissolved in THF (6 mL). Concentrated sulfuric acid (20 mL) was slowly added and the mixture was stirred at room temperature for 12 hours. The reaction mixture was poured into ice and extraction was performed with ethyl acetate. The organic layer was rinsed with aqueous saturated sodium hydrogencarbonate and then further rinsed with brine and dried over magnesium sulfate. The solvent was distilled off under reduced pressure, and the obtained solid was purified by silica-column chromatography (10/1 hexane/ethyl acetate (volume ratio)) to obtain compound F represented by formula (27) (122 mg, 2 steps, 62% yield) as a brown solid.

The evaluation results for the obtained compound F are as follows.
¹H NMR (400 MHz, CDCl₃) δ 0.77-0.94 (m, 3H), 1.17-1.33 (m, 8H), 2.60 (t, 2H, J = 7.8 Hz), 7.06 (s, 1H), 7.28 (s, 1H); MS (EI) m/z 433 (M⁺).

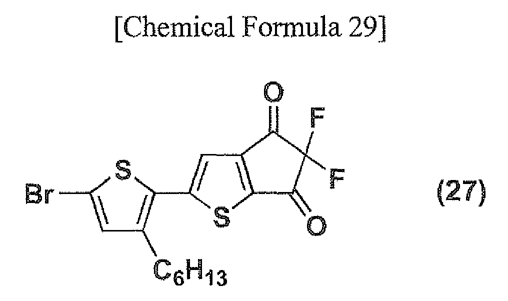

### (Synthesis Example 3)

After synthesizing compound G, it was used to synthesize compound H as an intermediate compound.

### <Synthesis of Compound G>

After placing 2-tributylstannylthiophene (4.27 g, 11.43 mmol) and 1,3,5-tribromobenzene (1.0 g, 3.18 mmol) in a 20 mL nitrogen-substituted two-necked flask, dry toluene (5 mL) was added.
After deaeration by bubbling, tetrakistriphenylphosphinepalladium(0) (92 mg, 0.080 mmol) was added and the mixture was heated to reflux for 12 hours. The solid was removed by Celite filtration, concentrated under reduced pressure and purified by column chromatography (silica gel, hexane/dichloromethane = 10/1 (volume ratio)) to obtain compound G represented by formula (28) (964 mg, 93% yield).

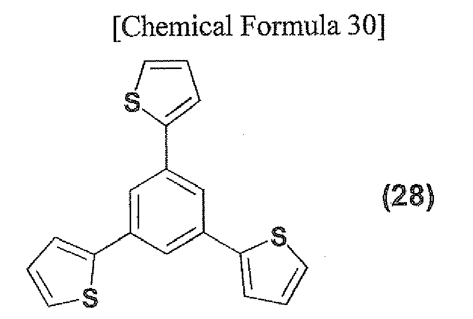

### <Synthesis of Compound H>

After placing compound G (964 mg, 2.97 mmol) in a 50 mL heat-dried, nitrogen-substituted two-necked flask and adding dry THF (10 mL), the mixture was cooled to -78°C and 1.6 M n-butyllithium/hexane (6.2 mL, 9.80 mmol) was added dropwise. After stirring for 30 minutes, tributyltin chloride (3.48 g, 10.69 mmol) was added in one portion. The obtained solution was warmed to room temperature and then stirred for 3 hours. Water (20 mL) and hexane (20 mL) were added to the obtained reaction mixture, and the organic layer was washed twice with water (20 mL) and dried over anhydrous magnesium sulfate. After removing off the insoluble portion by filtration, it was concentrated under reduced pressure and purified by column chromatography (alumina, hexane) to obtain compound H represented by formula (29) (2.33 g, 87% yield).

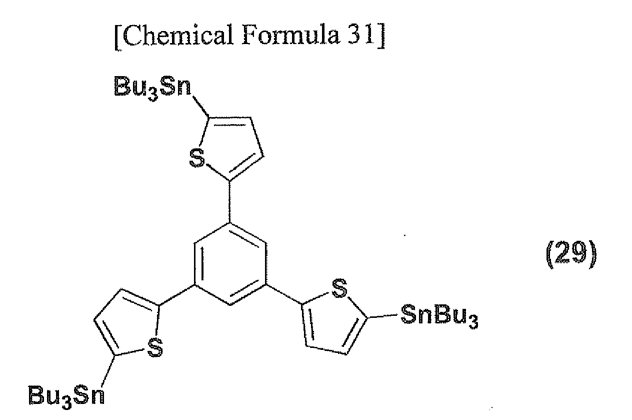

### (Example 1: Synthesis of branched compound)

### <Synthesis of Compound I>

After placing compound F (40 mg, 0.0926 mmol), compound H (28 mg, 0.0232 mmol) and tetrakis(triphenylphosphine)palladium(0) (3 mg, 0.00232 mmol) in a 50 mL volumetric flask, the mixture was dissolved in toluene (1 mL). The mixture was stirred at 120°C for 12 hours, and then allowed to cool at room temperature. The solvent was distilled off under reduced pressure, and the crude purified product was passed through silica-column chromatography (CHCl₃) and then purified by GPC (CHCl₃) to obtain compound I represented by formula (30) (9 mg, 3 1 % yield) as a branched compound.

The evaluation results for the obtained compound I are as follows.
TLC R_{f} = 0.55 (ethyl acetate:hexane = 2:1 (volume ratio)); ¹H NMR (400 MHz, CDCl₃) δ 0.88-0.99 (m, 9H), 1.10-1.44 (m, 18H), 1.50-1.69 (m, 6H), 2.80-2.90 (m, 6H), 7.18 (d, 3H, J = 3.2 Hz), 7.22 (s, 3H), 7.48 (s, 3H), 7.54 (d, 3H, J = 3.2 Hz), 7.74 (s, 3H).

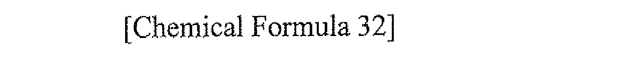

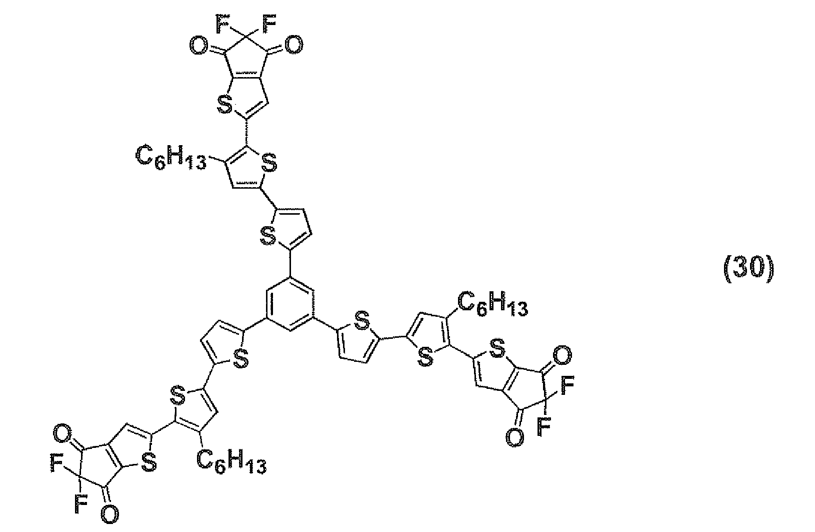

### (Example 2)

### <Fabrication of organic thin-film transistor and evaluation of transistor property>

A thermal oxidation film (silicon oxide film)-attached low resistance silicon wafer (gate electrode/insulating layer) was dipped in ethanol, distilled water and acetone in that order, and ultrasonic cleaning was performed. The silicon wafer was then subjected to UV-ozone cleaning to obtain a substrate with a hydrophilic surface. The substrate was dipped in hexamethyldisilazane:chloroform at room temperature and subjected to ultrasonic cleaning with chloroform to obtain a surface-treated substrate.

Next, a coating solution was prepared comprising compound I synthesized in Example 1 dissolved in chloroform. The solution was formed into a film by spin coating on a surface-treated substrate, to form an organic thin-film. Gold electrodes (source electrode, drain electrode) were formed on the organic thin-film by vacuum vapor deposition using a metal mask, to fabricate an organic thin-film transistor.

The obtained organic thin-film transistor was measured for organic transistor properties using a Semiconductor Parameter Analyzer (trade name "4200-SCS", by Keithley Instruments, Inc.), while varying the gate voltage Vg and source-drain voltage Vsd, and satisfactory n-type semiconductor Id-Vg characteristics were obtained. This indicated that the branched compound I has an excellent electron transport property.

### Reference Signs List

1: Substrate, 2: active layer, 2a: active layer, 3: insulating layer, 4: gate electrode, 5: source electrode, 6: drain electrode, 7a: first electrode, 7b: second electrode, 8: charge generation layer, 100: first embodiment of organic thin-film transistor, 110: second embodiment of organic thin-film transistor, 120: third embodiment of organic thin-film transistor, 130: fourth embodiment of organic thin-film transistor, 140: fifth embodiment of organic thin-film transistor, 150: sixth embodiment of organic thin-film transistor, 160: seventh embodiment of organic thin-film transistor, 200: embodiment of solar cell, 300: first embodiment of optical sensor, 310: second embodiment of optical sensor, 320: third embodiment of optical sensor.

## Claims

1. A branched compound having a construction with a core portion, at least 3 side chain portions bonded to the core portion, and end portions bonded to each of the side chain portions, wherein:
the side chain portions are groups in which a plurality of conjugated units are linked, at least one of the conjugated units being a divalent heterocyclic group,
at least one of the end portions is a group represented by formula (1), and
the side chain portions and the end portions are conjugated with the core portion. 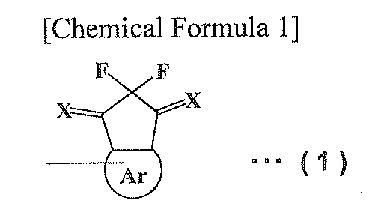 [In the formula, Ar represents an optionally substituted trivalent aromatic hydrocarbon or optionally substituted trivalent heterocyclic group, and X represents an oxygen atom, a sulfur atom, or a group represented by formula (a). When multiple X groups are present, they may be the same or different.] 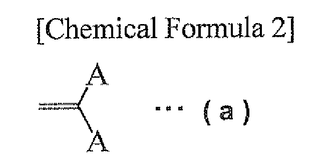 [In the formula, A represents hydrogen, a halogen atom or a monovalent group, and when multiple A groups are present, they may be the same or different, and at least one A is an electron-withdrawing group.]

2. The branched compound according to claim 1, wherein the group represented by formula (1) is a group represented by formula (2). 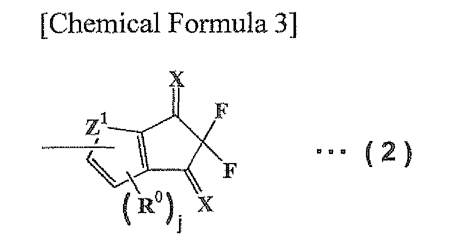 [In the formula, X has the same definition as above, R⁰ represents hydrogen or a monovalent group, and j is an integer from 1 to the number of substitutable sites on the ring to which R⁰ is bonded. When multiple R⁰ groups are present, they may be the same or different. Z¹ is any group represented by formula (i), (ii), (iii), (iv), (v), (vi), (vii), (viii) or (ix), among which R¹, R², R³ and R⁴ may be the same or different and each represents hydrogen or a monovalent group, and R¹ and R² may be bonded together to form a ring.]

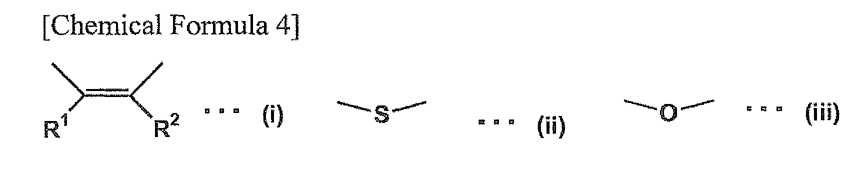

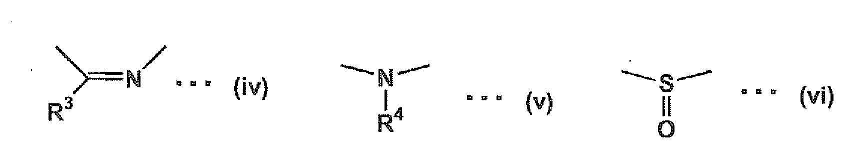

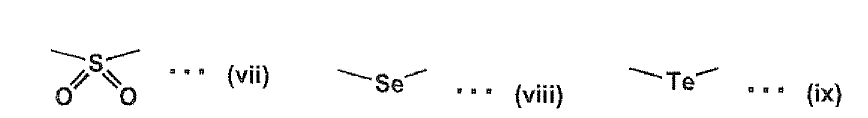

3. The branched compound according to claim 2, wherein Z¹ is a group represented by formula (ii).

4. The branched compound according to any one of claims 1 to 3, wherein the side chain portions are groups represented by formula (3). 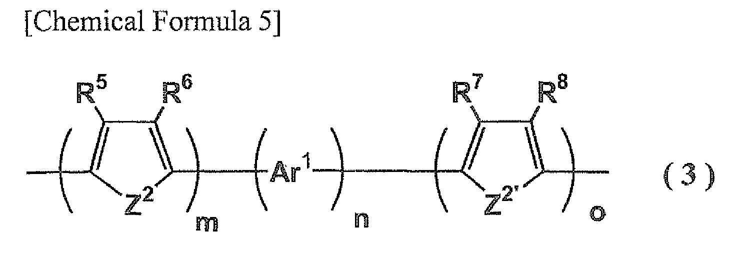 [In the formula, m, n and o are the same or different and each represents an integer of 0-10. This is with the proviso that m+o is an integer of 1 or greater. Ar¹ represents an optionally substituted divalent aromatic hydrocarbon or an optionally substituted divalent heterocyclic group, and R⁵, R⁶, R⁷ and R⁸ are the same or different and each represents hydrogen, alkyl, alkoxy, optionally substituted aryl or an optionally substituted monovalent heterocyclic group. The groups represented by Ar¹, R⁵, R⁶, R⁷ and R⁸ may have all or a portion of their hydrogens replaced by fluorine. Z² and Z²' are the same or different and each is a group represented by formula (xi), (xii), (xiii), (xiv), (xv), (xvi), (xvii), (xviii) or (xix), among which R⁹, R¹⁰, R¹¹ and R¹² are the same or different and each represents hydrogen or a monovalent group, and R⁹ and R¹⁰ may be bonded together to form a ring. When multiple Z², Z^{2'}, Ar¹, R⁵, R⁶, R⁷, R⁸, R⁹, R¹⁰, R¹¹ and R¹² groups are present, they may be the same or different.]

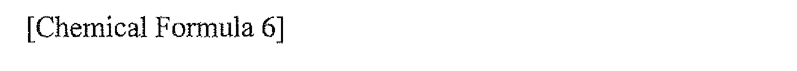

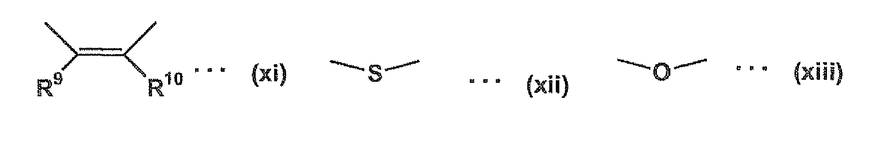

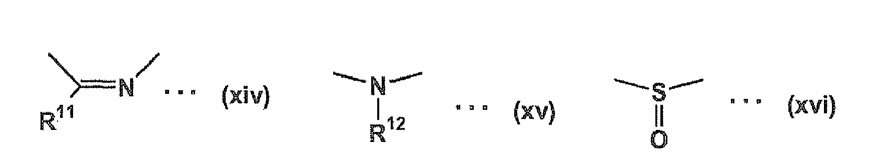

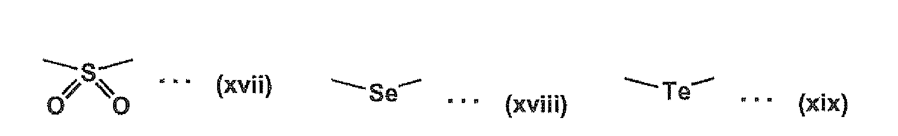

5. The branched compound according to claim 4, wherein either or both Z² and Z^{2'} are groups represented by formula (xii).

6. The branched compound according to any one of claims 1 to 5, wherein the core portion is any group represented by formula (I), (II), (III), (IV) or (V). 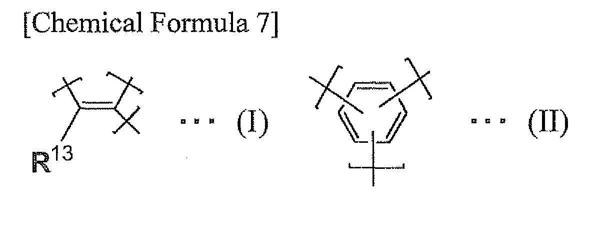 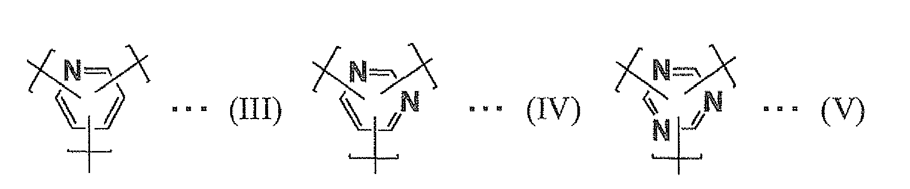 [In the formula, R¹³ represents hydrogen, alkyl, aryl or cyano.]

7. An organic thin-film comprising the branched compound according to any one of claims 1 to 6.

8. An organic thin-film element comprising the organic thin-film according to claim 7.

9. An organic thin-film transistor comprising the organic thin-film according to claim 7.

10. An organic solar cell comprising the organic thin-film according to claim 7.

11. An optical sensor comprising the organic thin-film according to claim 7.
